(19) Europäisches Patentamt
European Patent Office
Office européen des brevets

(11) EP 2 921 981 A2

(12) # EUROPÄISCHE PATENTANMELDUNG

(43) Veröffentlichungstag:
**23.09.2015 Patentblatt 2015/39**

(51) Int Cl.:
***G06F 19/22*** *(2011.01)*

(21) Anmeldenummer: **14197003.8**

(22) Anmeldetag: **09.12.2014**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **20.01.2014 DE 102014000682**

(71) Anmelder: **Hoelken, Horst**
**42579 Heiligenhaus (DE)**

(72) Erfinder: **Hoelken, Horst**
**42579 Heiligenhaus (DE)**

(74) Vertreter: **Fitzner, Uwe**
**Dres. Fitzner**
**Patent- und Rechtsanwälte**
**Hauser Ring 10**
**40878 Ratingen (DE)**

(54) **Verfahren zur Synthese hochspezieller Aminosäuresequenzen**

(57) Verfahren zur Synthese hochspezieller Aminosäuresequenzen basierend auf mathematischen Grundlagen und fachüblichen technischen Herstellverfahren, sowie Verfahren zur Überprüfung von Aminosäuresequenzen auf Genfehler mittels der hochspeziellen Aminosäuresequenzen.

EP 2 921 981 A2

## Beschreibung

Die vorliegende Anmeldung nimmt die Priorität der DE 10 2014 000 682.4 in Anspruch.

Das Prioritätsdokument ist durch Verweis vollumfänglich in die vorliegende Offenbarung einbezogen (= incorporated by reference in its entirety).

Alle in der vorliegenden Anmeldung zitierten Dokumente sind durch Verweis vollumfänglich in die vorliegende Offenbarung einbezogen (= incorporated by reference in their entirety).

Die vorliegende Erfindung betrifft Verfahren zur Synthese hochspezieller Aminosäuresequenzen basierend auf mathematischen Grundlagen und fachüblichen technischen Herstellverfahren, sowie Verfahren zur Überprüfung von natürlichen Aminosäuresequenzen auf Genfehler mittels der hochspeziellen Aminosäuresequenzen.

<u>Aufgabe:</u>

Aufgabe der vorliegenden Erfindung war es einen verlässlichen Weg zu finden, mit dem hochspezielle Aminosäuresequenzen gefunden werden können, die besonders gute reproduzierbare Eigenschaften aufweisen und verlässlich in gleicher Abfolge aufgebaut werden können.

Ferner war es Aufgabe, der vorliegenden Erfindung, ein Verfahren zu finden, mit dem es möglich ist, Genfehler in natürlich vorkommenden oder synthetisierten Aminosäuresequenzen aufzuspüren.

<u>Lösung:</u>

Diese Aufgabe wird durch die in den Ansprüchen und der nachfolgenden Beschreibung dargestellten Verfahren und Methoden gelöst.

<u>Detaillierte Beschreibung:</u>

Gegenstand der vorliegenden Erfindung ist ein Verfahren zur Synthese hochspezieller Aminosäuresequenzen umfassend die folgenden Schritte:

a) Erstellung der Adjazenzmatrizen 12. Ordnung von den Nucleotidbasen,

b) Nichtkommutative Matrizenmultiplikation mit jeweils 3 Adjazenzmatrizen 12. Ordnung

c) Durchführen eine Ordnungserniedrigung nach Schur mit jeweils einer "Codon-Matrix" 12. Ordnung zur Erstellung von "Codon-Matrizen" 6. Ordnung,

d) Durchführen der Allgemeinen Eigenwertaufgabe für Matrizenpaare mit jeweils 2 "Codon-Matrizen" 6. Ordnung, wobei je "Codonpaar-Matrix" ein "6-Eigenwerte-Satz" mit 6 reellen und (oder) konjugiert-komplexen Eigenwerten existiert,

e) bei festgelegtem ersten Codonpaar, in Verbindung mit dem "Startcodon", mit einem reellen Eigenwert für den Aufbau einer Sequenz Suchen von Codonpaaren, die dann eine "wechselseitige Eigenwerte-Invarianz" aufweisen,

f) Übersetzung der "Codonpaar-Sequenz" in die entsprechende Aminosäuresequenz und

g) Herstellung der resultierenden Aminosäuresequenz mit fachüblichen Verfahren, bevorzugt mittels Merrifield-Synthese,

h) gegebenenfalls Überprüfung der biochemischen Funktion im Anschluss an deren Synthese mittels fachüblicher Methoden.

Anstelle der Merrifield-Synthese können die Aminosäuresequenzen selbstverständlich auch über gen- oder biotechnologische Verfahren hergestellt werden.

Ferner ist Gegenstand der vorliegenden Erfindung ein erstes Verfahren zur Überprüfung von natürlichen oder synthetisch hergestellten Aminosäuresequenzen auf (Gen-) Fehler bei dem

Ia) mit einem Verfahren gemäß der Schritte a) bis g) eine Aminosäuresequenz *synthetisiert* wird,

II) die zu überprüfende Aminosäuresequenz herangezogen wird,

III) die beiden Sequenzen verglichen werden und

IV) anhand der Abweichungen der beiden Sequenzen eventuelle Fehler bestimmt werden.

Die zu überprüfende Aminosäuresequenz kann im Rahmen dieses ersten Verfahrens zur Überprüfung auf Genfehler entweder bereits eine bekannte Sequenz sein oder aus zu überprüfenden Proteinen mittels Proteinsequenzierung, bevorzugt über Edman-Abbau oder De-Novo-Sequenzierung, gewonnen werden.

Bei dem Verfahren gemäß der Schritte a) bis g) beginnt man mit demselben Aminosäurepaar, das bei der zu überprüfenden Aminosäuresequenz am Anfang steht.

Ein entsprechendes zweites Verfahren zur Überprüfung von natürlichen oder synthetisch hergestellten Aminosäuresequenzen auf (Gen-) Fehler bei dem

Ib) mit einem Verfahren gemäß der Schritte a) bis f) eine Aminosäuresequenz *errechnet* wird,

II) die zu überprüfende Aminosäuresequenz, hergestellt, insbesondere aus Proteinen, mittels fachüblicher Verfahren, herangezogen wird,

III) die beiden Sequenzen verglichen werden und

IV) anhand der Abweichungen der beiden Sequenzen eventuelle Fehler bestimmt werden,

ist ebenfalls Gegenstand der vorliegenden Erfindung.

Die zu überprüfende Aminosäuresequenz kann im Rahmen dieses zweiten Verfahrens zur Überprüfung auf Genfehler aus zu überprüfenden Proteinen mittels Proteinsequenzierung, bevorzugt über Edman-Abbau oder De-Novo-Sequenzierung, gewonnen werden.

Bei dem Verfahren gemäß der Schritte a) bis f) beginnt man mit demselben Aminosäurepaar, das bei der zu überprüfenden Aminosäuresequenz am Anfang steht.

Zum Vergleich der Sequenzen werden diese in Form Ihrer schriftlichen Darstellung, oder bevorzugt im Computer mit üblichen Programmen, miteinander verglichen.

Sofern man bei den beiden vorgenannten Verfahren zur Überprüfung von natürlichen oder synthetisch hergestellten Aminosäuresequenzen auf (Gen-) Fehler bei der Berechnung als jeweils nächstfolgende Aminosäure mehrere erhalten kann, ist hier eine Abweichung bzw. ein Fehler im Sinne der vorliegende Erfindung natürlich nur dann zu sehen, wenn keine diese mehreren Möglichkeiten mit der entsprechenden Aminosäure der zu überprüfenden Sequenz übereinstimmt.

Stimmt eine berechnete Aminosäure mit der betreffenden der zu überprüfenden Sequenz überein, so liegt hier kein Fehler vor und es wird ausgehend von dieser dann die Berechnung fortgesetzt.

Die vorliegende Erfindung betrifft die Synthese von hochspeziellen Aminosäuresequenzen auf Basis spezieller (mathematischer) Überlegungen zur Begründung der Primärstruktur bei Aminosäuresequenzen.

Die 4 Nucleotidbasen werden bei den zur Anwendung gelangenden Überlegungen als spezielle "Adjazenzmatrizen" "abgebildet", unter der Voraussetzung, dass - in mathematischer Hinsicht - keine "Degeneration" im Genetischen Code vorliegt.

Das wichtigste mathematische Grundprinzip ist die "nichtkommutative Matrizenmultiplikation" (zum Teil mit NkMM abgekürzt). Hierzu seien einige wenige Beispiele genannt: CAU, ACU, UCA, also die Codons für die Aminosäuren: Histidin, Threonin und Serin, sind voneinander verschiedene Matrizen, obwohl die "Menge": C, A, U stets die gleiche ist. Sehr wichtig ist hier - in mathematischer Hinsicht - die äquivalente Bedeutung der jeweils dritten Nucleotidbasen im Codon, die jeweils dritte Nucleotidbase tritt damit aus dem "lehrbuchhaften Schattendasein" heraus.

Selbstverständlich ist es nicht erlaubt, anzunehmen, dass die Matrizenprodukte 12. oder 6. Ordnung ebenfalls "Adjazenzmatrizen" wären, wichtig ist nur, dass letztendlich jedes der 64 Codons eine für sich charakteristische Matrix besitzt, wobei jeweils die gleichen "Ausgangsmatrizen" und dieselben Rechenregeln der Matrizenrechnung eingesetzt bzw. eingehalten werden.

Eine rein mathematische Bedeutung hat auch die Bildung von Codonpaaren; nur aus Gründen einer "vorgezogenen Spezifität" wird mit diesen Codonpaaren gearbeitet. Die Bildung der Schur-Complemente und der Einsatz von Codonpaaren "vereinfacht" enorm das gesamte algorithmische Rechenverfahren.

Die Allgemeine Eigenwertaufgabe wird auf die spezielle Eigenwertaufgabe zurückgeführt. Bei den Eigenwerten selbst sind die Übereinstimmung bzw. Gleichheit wesentlich, die Zahlengrößen selbst sind weniger relevant. Es wird also eine Art "Invariantentheorie für Polycodonpaarsequenzen" (also stets ohne Berücksichtigung der "Ribose-Phosphat-Brücken" der RNA) geschaffen.

Sowohl bei der A-Kette als auch bei der B-Kette des Humaninsulins findet man, nach Übersetzung in alle nur möglichen "Codonpaar-Sequenzen", eine "wechselseitige Eigenwerte-Invarianz" ohne und mit Vorzeichenwechsel bei den Eigenwerten. Diese "wechselseitige Eigenwerte-Invarianz ist das Kernstück der dieser Erfindung zugrundeliegenden Überlegungen, sie kann für den Aufbau künstlicher Aminosäuresequenzen benutzt werden. Kurz gesagt: 2 beliebige Zahlreihen (beispielsweise Zahlen zwischen 1 und 20) mit Eigenwerte-Invarianz werden als "Codonpaar-Sequenz" "abgebildet", dann wird in die entsprechende Aminosäuresequenz übersetzt. Um Mehrdeutigkeiten auszuschalten, wird eine "Codonpaar-Sequenz" ohne Vorzeichenwechsel bei den Eigenwerten mit einer zweiten, "degeneriert" zur ersten, "Codonpaar-Sequenz" mit Vorzeichenwechsel bei den Eigenwerten "abgesichert".

Bei der genetischen Übersetzung der mit der "wechselseitigen Eigenwerte-Invarianz" aufgefundenen "Polycodonpaarsequenz" (und damit der entsprechenden Polynucleotidsequenz) durch die bekannten gentechnischen Verfahren, z.B. in die genetisch entsprechende Aminosäuresequenz, geht die hier angewandte Mathematik "verloren", aber genau an diesem Punkt erkennt man überraschenderweise, dass die der vorliegenden Erfindung zugrundeliegenden Überlegungen dazu dienten, dass das jeweils "sequenzgerechte" Codonpaar und damit das entsprechende Aminosäurepaar zur Auswahl kam.

Durch diese "wechselseitige Eigenwerte-Invarianz" kann man eine Steuerung, unter Vermittlung der Codonpaare, der "relativ richtigen" Fortführung oder des Anwachsens der Aminosäuresequenz und damit das Aussehen der Primärstruktur der Proteinkette, erreichen.

Dann kann man, nach Kenntnis einer derart aufgefundenen Aminosäuresequenz, dieselbe Sequenz ohne irgendwelche Eigenwerte-Invarianz formulieren, dafür muss sie aber schon bekannt sein; das Kausalitätsprinzip fordert dies.

Zueinander "degenerierte" Codonpaare treten aufgrund ihrer voneinander verschiedenen Eigenwerte an ganz bestimmten Positionen einer Sequenz auf, und ermöglichen somit die Wiederholung des entsprechenden (gleichen) Aminosäurepaares. Dies alles ist letztendlich auf den Einfluss der jeweils dritten Nucleotidbase des Codons zurückzuführen.

Bemerkenswert ist noch, dass Eigenwerte von Codonpaar-Matrizen auftreten, die auch Zahlen des Goldenen Schnitts sind, GS-Zahlen: 0.382, 0.618, (1), 1.618, 2.618. Mit der "Balmerschen Zahlenbeziehung" können GS-Zahlen in Eigenwerte von Codonpaar-Matrizen umgerechnet werden.

Es ist also im Rahmen der vorliegenden Erfindung auf dieser Basis möglich, eine Aminosäuresequenz zu schaffen, deren "Codonpaar-Sequenz" und damit deren "Codonpaar-Matrizen" die ganze Sequenz hindurch eine direkte Eigenwerte-Invarianz mit Zahlen des Goldenen Schnitts aufweist, auch mit und ohne Vorzeichenwechsel.

Unter der Voraussetzung, dass sich beim Aufbau einer Aminosäuresequenz Aminosäurepaare und bei bereits vorhandenen Aminosäurepaaren die dazu inversen Paare nicht wiederholen, ist die "Länge" einer Aminosäuresequenz "vorausbestimmt" und letztendlich abhängig vom ersten Aminosäurepaar.

Der Begriff (Gen-)Fehler im Sinne der vorliegenden Erfindung bedeutet insbesondere Abweichungen einer zu untersuchenden natürlichen oder synthetisierten Aminosäuresequenz von einer Aminosäuresequenz, die mit der in der vorliegenden Erfindung beschriebenen Methode über die Eigenwertematrizen der zugehörigen Codons bzw. Codonpaare berechnet wurde, beispielsweise ein Fehlen bzw. die Unterbrechung einer wechselseitigen Eigenwerteinvarianz.

Ein Beispiel hierzu: in einer genetisch intakten RNA tritt (in Verbindung mit einem anderen Codon X) das Codon CCC für Prolin auf. Wenn nun das C in der Mitte durch z.B. eine Spontanmutation zu Uracil wird (oxidative Desaminierung von Cytosin zu Uracil), dann erhält man als Codon CUC, das Codon für Leucin. In der entsprechenden Aminosäuresequenz steht also an der Stelle von prolin das leucin. Annahme: das zweite Codon ist GAA oder GAG für glu. Für mindestens zwei der folgenden 8 Codonpaare existiert also in der intakten RNA eine "wechselseitige Eigenwerte-Invarianz" mit den direkt benachbarten Codonpaaren in der RNA: CCU-GAA, CCC-GAA, CCA-GAA, CCG-GAA, CCU-GAG, CCC-GAG, CCA-GAG, CCG-GAG. Nach Spontanmutation wäre aber jetzt vorhanden: CUU-GAA, CUC-GAA, CUA-GAA, CUG-GAA, CUU-GAG, CUC-GAG, CUA-GAG, CUG-GAG. Wenn für alle diese letzten 8 Codonpaare keine Eigenwerteinvarianz mit den Nachbarn zustande kommt, liegt ein "Genfehler" vor.

Hierbei ist zunächst nicht direkt von einer Aminosäuresequenz die Rede, jedoch lässt sich eine solche aus den Codons selbstverständlich ohne weiteres erhalten.

Entsprechend können durch die vorliegende Erfindung Fehler in der RNA - aber letztlich auch in der DNA - Genfehler festgestellt werden (wobei die Introns der untersuchten RNA bekannt sein müssen).

Wenn man also beispielsweise ein natürliche Aminosäuresequenz überprüfen möchte, ob Genfehler vorliegen bzw. ob alle Aminosäureradikale am "richtigen" Platz stehen, kann man wie folgt vorgehen: man übersetzt diese Aminosäuresequenz in alle nur möglichen Codonpaarsequenzen. Wenn dann an irgendeiner Stelle der Sequenz die wechselseitige Eigenwerte-Invarianz unterbrochen ist (also nicht existiert), dann steht an der jeweils betreffenden Stelle der Aminosäuresequenz eine falsche Aminosäure, also muss ein Genfehler in der entsprechenden RNA vorhanden gewesen sein.

Zur Veranschaulichung werden die bei der vorliegenden Erfindung zur Anwendung gelangenden Überlegungen

im Folgenden als Einfügung der entsprechenden Veröffentlichung dargestellt.

*Beginn Einschub:*

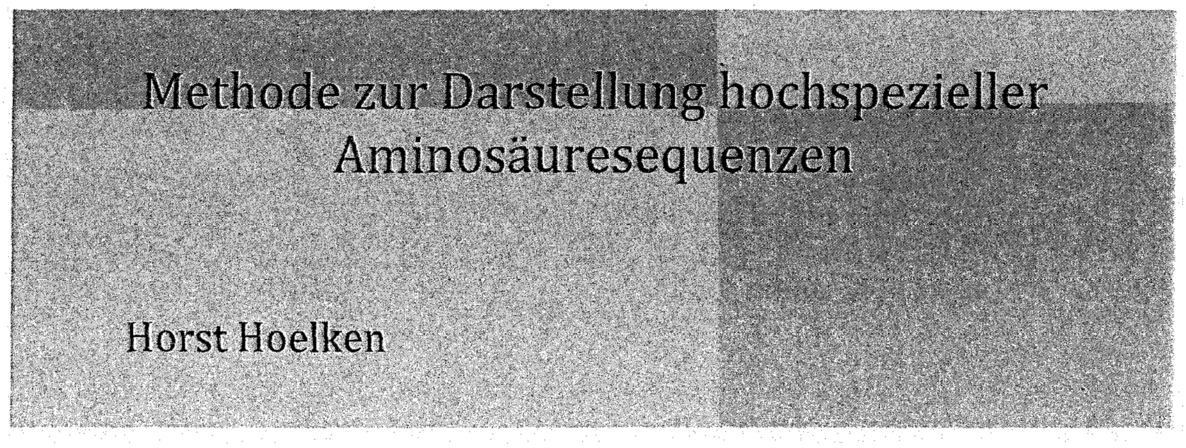


**Inhaltsverzeichnis**

| | |
|---|---|
| EINLEITUNG | 4 |
| DIE *DRITTE* NUCLEOTIDBASE DES CODONS | 6 |
| DIE EIGENWERT-AUFGABE | 6 |
| DER NEGATIVE EIGENWERT | 7 |
| "CODONPAAR-MATRIZEN" MIT BESONDEREN EIGENWERTEN | 8 |
| DAS GLEICHNAMIGE AMINOSÄUREPAAR | 9 |
| "PROSYMMETRIE" UND "NICHTSYMMETRIE" BEI AMINOSÄUREPAAREN | 9 |
| DIE "WECHSELSEITIGE EIGENWERTE-INVARIANZ" | 9 |
| DIE "WECHSELSEITIGE EIGENWERTE-INVARIANZ" BEI "POLYCODONPAAR-SEQUENZEN". ALLGEMEINES SCHEMA | 10 |
| A D C B F | 10 |
| A’ D’ C’ B’ F’ | 10 |
| DIE "NICHTPOLARE" UND "POLARE" "WECHSELSEITIGE EIGENWERTE- INVARIANZ" BEIM NONAPEPTID BRADYKININ UND DER A- UND B-KETTE DES HUMANINSULINS. TABELLEN | 11 |
| METHODEN ZUR DARSTELLUNG HOCHSPEZIELLER AMINOSÄURESEQUENZEN | 14 |
| *A. ALLGEMEINE EINFÜHRUNG IN DIE PROBLEMSTELLUNG* | 14 |
| *B. DIE FESTLEGUNG EINER EIGENWERTKLASSE* | 14 |
| *C. AMINOSÄURESEQUENZEN MIT BERÜCKSICHTIGUNG SYMMETRISCHER ASPEKTE.* | 15 |
| *D. DIE PERMUTATIONSMETHODE* | 15 |
| E. *AMINOSÄURESEQUENZEN UND DIE ZAHLEN DES GOLDENEN SCHNITTES* | 16 |
| "GENFEHLER" ? | 17 |
| EIGENWERTE, BALMER-FORMEL, ZAHLEN DES GOLDENEN SCHNITTES | 17 |
| ZUSAMMENFASSUNG UND AUSBLICK | 18 |
| LITERATURVERZEICHNIS | 20 |

(fortgesetzt)


| | |
|---|---|
| ANHANG | 21 |
| DIE SCHUR-COMPLEMENTE DER 64 CODONS | 21 |


## EINLEITUNG

Ursprünglich sollten Aminosäuresequenzen - Peptide, Proteine - graphen- und matrizentheoretisch untersucht werden, um festzustellen, ob das Vorliegen einer besonderen biochemischen Funktion dieser Sequenzen auf eine "mathematische Invariantentheorie" zurückgeführt werden könnte. Es bereitete jedoch besondere Schwierigkeiten, für die 20 Aminosäuren(-radikale) eine *einheitliche* Matrizenordnung festzulegen, so dass man - unter Heranziehung des *Genetischen Codes* [1)] - im Sinne der Aufgabenstellung in einem "matrizentheoretischen Umweg" letztendlich mit *Adjazenzmatrizen* [2)] der vier Nucleotidbasen arbeitete. Bei diesen *Adjazenzmatrizen* werden nur die Zahlen 1 und 0 benutzt; *minus* 1 bedeutet: der (virtuelle) Pfeil beim "exocyclischen Substituenten" zeigt - unabhängig von der Nummerierung - zum Ringatom hin. Ganz allgemein dient das Wasserstoff-Atom nur zur Vervollständigung der Matrizenordnung 12, kein H-Atom wird also irgendwie " bevorzugt". Die Entwicklung und Formulierung der "Codon-Matrizen" von 12. und 6. Ordnung erfolgt stets durch *die "nichtkommutative Matrizenmultiplikation"* [3)].

| *ADENIN* (A) | *CYTOSIN*(C) |
|---|---|
| 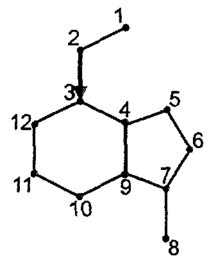 $\begin{pmatrix} 0 & 1 & 0 & 0 & 0 & 0 & 0 & 0 & 0 & 0 & 0 & 0 \\ 1 & 0 & -1 & 0 & 0 & 0 & 0 & 0 & 0 & 0 & 0 & 0 \\ 0 & 1 & 0 & 1 & 0 & 0 & 0 & 0 & 0 & 0 & 0 & 1 \\ 0 & 0 & 1 & 0 & 1 & 0 & 0 & 0 & 1 & 0 & 0 & 0 \\ 0 & 0 & 0 & 1 & 0 & 1 & 0 & 0 & 0 & 0 & 0 & 0 \\ 0 & 0 & 0 & 0 & 1 & 0 & 1 & 0 & 0 & 0 & 0 & 0 \\ 0 & 0 & 0 & 0 & 0 & 1 & 0 & 1 & 1 & 0 & 0 & 0 \\ 0 & 0 & 0 & 0 & 0 & 0 & 1 & 0 & 0 & 0 & 0 & 0 \\ 0 & 0 & 0 & 1 & 0 & 0 & 1 & 0 & 0 & 1 & 0 & 0 \\ 0 & 0 & 0 & 0 & 0 & 0 & 0 & 0 & 1 & 0 & 1 & 0 \\ 0 & 0 & 0 & 0 & 0 & 0 & 0 & 0 & 0 & 1 & 0 & 1 \\ 0 & 0 & 1 & 0 & 0 & 0 & 0 & 0 & 0 & 0 & 1 & 0 \end{pmatrix}$ | 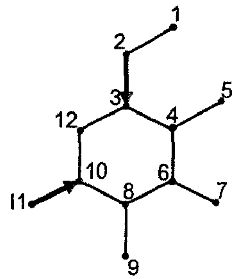 $\begin{pmatrix} 0 & 1 & 0 & 0 & 0 & 0 & 0 & 0 & 0 & 0 & 0 & 0 \\ 1 & 0 & -1 & 0 & 0 & 0 & 0 & 0 & 0 & 0 & 0 & 0 \\ 0 & 1 & 0 & 1 & 0 & 0 & 0 & 0 & 0 & 0 & 0 & 1 \\ 0 & 0 & 1 & 0 & 1 & 1 & 0 & 0 & 0 & 0 & 0 & 0 \\ 0 & 0 & 0 & 1 & 0 & 0 & 0 & 0 & 0 & 0 & 0 & 0 \\ 0 & 0 & 0 & 1 & 0 & 0 & 1 & 1 & 0 & 0 & 0 & 0 \\ 0 & 0 & 0 & 0 & 0 & 1 & 0 & 0 & 0 & 0 & 0 & 0 \\ 0 & 0 & 0 & 0 & 0 & 1 & 0 & 0 & 1 & 1 & 0 & 0 \\ 0 & 0 & 0 & 0 & 0 & 0 & 0 & 1 & 0 & 0 & 0 & 0 \\ 0 & 0 & 0 & 0 & 0 & 0 & 0 & 1 & 0 & 0 & 1 & 1 \\ 0 & 0 & 0 & 0 & 0 & 0 & 0 & 0 & 0 & -1 & 0 & 0 \\ 0 & 0 & 1 & 0 & 0 & 0 & 0 & 0 & 0 & 1 & 0 & 0 \end{pmatrix}$ |

Die **"Punkt-Strich-Formeln" und Adjazenzmatrizen** (Adjazent, Duden = der Anrainer, Grenznachbar) der vier **Nucleotidbasen** *ADENIN, CYTOSIN, GUANIN, URACIL* (Thymin wäre hier - rein mathematisch - auch Uracil).

| *GUANIN* (G) | | *URACIL* (U) | |
|---|---|---|---|
| 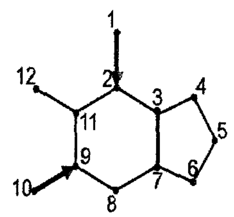 | $\begin{pmatrix} 0 & -1 & 0 & 0 & 0 & 0 & 0 & 0 & 0 & 0 & 0 & 0 \\ 1 & 0 & 1 & 0 & 0 & 0 & 0 & 0 & 0 & 0 & 1 & 0 \\ 0 & 1 & 0 & 1 & 0 & 0 & 1 & 0 & 0 & 0 & 0 & 0 \\ 0 & 0 & 1 & 0 & 1 & 0 & 0 & 0 & 0 & 0 & 0 & 0 \\ 0 & 0 & 0 & 1 & 0 & 1 & 0 & 0 & 0 & 0 & 0 & 0 \\ 0 & 0 & 0 & 0 & 1 & 0 & 1 & 0 & 0 & 0 & 0 & 0 \\ 0 & 0 & 1 & 0 & 0 & 1 & 0 & 1 & 0 & 0 & 0 & 0 \\ 0 & 0 & 0 & 0 & 0 & 0 & 1 & 0 & 1 & 0 & 0 & 0 \\ 0 & 0 & 0 & 0 & 0 & 0 & 0 & 1 & 0 & 1 & 1 & 0 \\ 0 & 0 & 0 & 0 & 0 & 0 & 0 & 0 & -1 & 0 & 0 & 0 \\ 0 & 1 & 0 & 0 & 0 & 0 & 0 & 0 & 1 & 0 & 0 & 1 \\ 0 & 0 & 0 & 0 & 0 & 0 & 0 & 0 & 0 & 0 & 1 & 0 \end{pmatrix}$ | 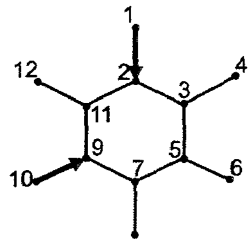 | $\begin{pmatrix} 0 & -1 & 0 & 0 & 0 & 0 & 0 & 0 & 0 & 0 & 0 & 0 \\ 1 & 0 & 1 & 0 & 0 & 0 & 0 & 0 & 0 & 0 & 1 & 0 \\ 0 & 1 & 0 & 1 & 1 & 0 & 0 & 0 & 0 & 0 & 0 & 0 \\ 0 & 0 & 1 & 0 & 0 & 0 & 0 & 0 & 0 & 0 & 0 & 0 \\ 0 & 0 & 1 & 0 & 0 & 1 & 1 & 0 & 0 & 0 & 0 & 0 \\ 0 & 0 & 0 & 0 & 1 & 0 & 0 & 0 & 0 & 0 & 0 & 0 \\ 0 & 0 & 0 & 0 & 1 & 0 & 0 & 1 & 1 & 0 & 0 & 0 \\ 0 & 0 & 0 & 0 & 0 & 0 & 1 & 0 & 0 & 0 & 0 & 0 \\ 0 & 0 & 0 & 0 & 0 & 0 & 1 & 0 & 0 & 1 & 1 & 0 \\ 0 & 0 & 0 & 0 & 0 & 0 & 0 & 0 & -1 & 0 & 0 & 0 \\ 0 & 1 & 0 & 0 & 0 & 0 & 0 & 0 & 1 & 0 & 0 & 1 \\ 0 & 0 & 0 & 0 & 0 & 0 & 0 & 0 & 0 & 0 & 1 & 0 \end{pmatrix}$ |

**Das Matrizenprodukt UAC 12. Ordnung, die (musterhafte) Entwicklung des Schur-Complements 6. Ordnung von UAC, das Codon für die Aminosäure Tyrosin, abgekürzt: tyr.**

$$\underset{\textbf{U}}{\begin{pmatrix} 0 & -1 & 0 & 0 & 0 & 0 & 0 & 0 & 0 & 0 & 0 & 0 \\ 1 & 0 & 1 & 0 & 0 & 0 & 0 & 0 & 0 & 0 & 1 & 0 \\ 0 & 1 & 0 & 1 & 1 & 0 & 0 & 0 & 0 & 0 & 0 & 0 \\ 0 & 0 & 1 & 0 & 0 & 0 & 0 & 0 & 0 & 0 & 0 & 0 \\ 0 & 0 & 1 & 0 & 0 & 1 & 1 & 0 & 0 & 0 & 0 & 0 \\ 0 & 0 & 0 & 0 & 1 & 0 & 0 & 0 & 0 & 0 & 0 & 0 \\ 0 & 0 & 0 & 0 & 1 & 0 & 0 & 1 & 1 & 0 & 0 & 0 \\ 0 & 0 & 0 & 0 & 0 & 0 & 1 & 0 & 0 & 0 & 0 & 0 \\ 0 & 0 & 0 & 0 & 0 & 0 & 1 & 0 & 0 & 1 & 1 & 0 \\ 0 & 0 & 0 & 0 & 0 & 0 & 0 & 0 & -1 & 0 & 0 & 0 \\ 0 & 1 & 0 & 0 & 0 & 0 & 0 & 0 & 1 & 0 & 0 & 1 \\ 0 & 0 & 0 & 0 & 0 & 0 & 0 & 0 & 0 & 0 & 1 & 0 \end{pmatrix}} \circ \underset{\textbf{A}}{\begin{pmatrix} 0 & 1 & 0 & 0 & 0 & 0 & 0 & 0 & 0 & 0 & 0 & 0 \\ 1 & 0 & -1 & 0 & 0 & 0 & 0 & 0 & 0 & 0 & 0 & 0 \\ 0 & 1 & 0 & 1 & 0 & 0 & 0 & 0 & 0 & 0 & 0 & 1 \\ 0 & 0 & 1 & 0 & 1 & 0 & 0 & 0 & 1 & 0 & 0 & 0 \\ 0 & 0 & 0 & 1 & 0 & 1 & 0 & 0 & 0 & 0 & 0 & 0 \\ 0 & 0 & 0 & 0 & 1 & 0 & 1 & 0 & 0 & 0 & 0 & 0 \\ 0 & 0 & 0 & 0 & 0 & 1 & 0 & 1 & 1 & 0 & 0 & 0 \\ 0 & 0 & 0 & 0 & 0 & 0 & 1 & 0 & 0 & 0 & 0 & 0 \\ 0 & 0 & 0 & 1 & 0 & 0 & 1 & 0 & 0 & 1 & 0 & 0 \\ 0 & 0 & 0 & 0 & 0 & 0 & 0 & 0 & 1 & 0 & 1 & 0 \\ 0 & 0 & 0 & 0 & 0 & 0 & 0 & 0 & 0 & 1 & 0 & 1 \\ 0 & 0 & 1 & 0 & 0 & 0 & 0 & 0 & 0 & 0 & 1 & 0 \end{pmatrix}} \rightarrow \underset{\textbf{UA}}{\begin{pmatrix} -1 & 0 & 1 & 0 & 0 & 0 & 0 & 0 & 0 & 0 & 0 & 0 \\ 0 & 2 & 0 & 1 & 0 & 0 & 0 & 0 & 0 & 1 & 0 & 2 \\ 1 & 0 & 0 & 1 & 1 & 1 & 0 & 0 & 1 & 0 & 0 & 0 \\ 0 & 1 & 0 & 1 & 0 & 0 & 0 & 0 & 0 & 0 & 0 & 1 \\ 0 & 1 & 0 & 1 & 1 & 1 & 1 & 1 & 1 & 0 & 0 & 1 \\ 0 & 0 & 0 & 1 & 0 & 1 & 0 & 0 & 0 & 0 & 0 & 0 \\ 0 & 0 & 0 & 2 & 0 & 1 & 2 & 0 & 0 & 1 & 0 & 0 \\ 0 & 0 & 0 & 0 & 0 & 1 & 0 & 1 & 1 & 0 & 0 & 0 \\ 0 & 0 & 0 & 0 & 0 & 1 & 0 & 1 & 2 & 1 & 1 & 1 \\ 0 & 0 & 0 & -1 & 0 & 0 & -1 & 0 & 0 & -1 & 0 & 0 \\ 1 & 0 & 0 & 1 & 0 & 0 & 1 & 0 & 0 & 1 & 1 & 0 \\ 0 & 0 & 0 & 0 & 0 & 0 & 0 & 0 & 0 & 1 & 0 & 1 \end{pmatrix}} = \textbf{UA}$$

$$\underset{\textbf{UA}}{\begin{pmatrix} -1 & 0 & 1 & 0 & 0 & 0 & 0 & 0 & 0 & 0 & 0 & 0 \\ 0 & 2 & 0 & 1 & 0 & 0 & 0 & 0 & 0 & 1 & 0 & 2 \\ 1 & 0 & 0 & 1 & 1 & 1 & 0 & 0 & 1 & 0 & 0 & 0 \\ 0 & 1 & 0 & 1 & 0 & 0 & 0 & 0 & 0 & 0 & 0 & 1 \\ 0 & 1 & 0 & 1 & 1 & 1 & 1 & 1 & 1 & 0 & 0 & 1 \\ 0 & 0 & 0 & 1 & 0 & 1 & 0 & 0 & 0 & 0 & 0 & 0 \\ 0 & 0 & 0 & 2 & 0 & 1 & 2 & 0 & 0 & 1 & 0 & 0 \\ 0 & 0 & 0 & 0 & 0 & 1 & 0 & 1 & 1 & 0 & 0 & 0 \\ 0 & 0 & 0 & 0 & 0 & 1 & 0 & 1 & 2 & 1 & 1 & 1 \\ 0 & 0 & 0 & -1 & 0 & 0 & -1 & 0 & 0 & -1 & 0 & 0 \\ 1 & 0 & 0 & 1 & 0 & 0 & 1 & 0 & 0 & 1 & 1 & 0 \\ 0 & 0 & 0 & 0 & 0 & 0 & 0 & 0 & 0 & 1 & 0 & 1 \end{pmatrix}} \circ \underset{\textbf{C}}{\begin{pmatrix} 0 & 1 & 0 & 0 & 0 & 0 & 0 & 0 & 0 & 0 & 0 & 0 \\ 1 & 0 & -1 & 0 & 0 & 0 & 0 & 0 & 0 & 0 & 0 & 0 \\ 0 & 1 & 0 & 1 & 0 & 0 & 0 & 0 & 0 & 0 & 0 & 1 \\ 0 & 0 & 1 & 0 & 1 & 1 & 0 & 0 & 0 & 0 & 0 & 0 \\ 0 & 0 & 0 & 1 & 0 & 0 & 0 & 0 & 0 & 0 & 0 & 0 \\ 0 & 0 & 0 & 1 & 0 & 0 & 1 & 1 & 0 & 0 & 0 & 0 \\ 0 & 0 & 0 & 0 & 0 & 1 & 0 & 0 & 0 & 0 & 0 & 0 \\ 0 & 0 & 0 & 0 & 0 & 1 & 0 & 0 & 1 & 1 & 0 & 0 \\ 0 & 0 & 0 & 0 & 0 & 0 & 0 & 1 & 0 & 0 & 0 & 0 \\ 0 & 0 & 0 & 0 & 0 & 0 & 0 & 1 & 0 & 0 & 1 & 1 \\ 0 & 0 & 0 & 0 & 0 & 0 & 0 & 0 & 0 & -1 & 0 & 0 \\ 0 & 0 & 1 & 0 & 0 & 0 & 0 & 0 & 0 & 1 & 0 & 0 \end{pmatrix}} \rightarrow \underset{\textbf{UAC}}{\begin{pmatrix} 0 & 0 & 0 & 1 & 0 & 0 & 0 & 0 & 0 & 0 & 0 & 1 \\ 2 & 0 & 1 & 0 & 1 & 1 & 0 & 1 & 0 & 2 & 1 & 1 \\ 0 & 1 & 1 & 2 & 1 & 1 & 1 & 2 & 0 & 0 & 0 & 0 \\ 1 & 0 & 1 & 0 & 1 & 1 & 0 & 0 & 0 & 1 & 0 & 0 \\ 1 & 0 & 1 & 2 & 1 & 3 & 1 & 2 & 1 & 2 & 0 & 0 \\ 0 & 0 & 1 & 1 & 1 & 1 & 1 & 1 & 0 & 0 & 0 & 0 \\ 0 & 0 & 2 & 1 & 2 & 4 & 1 & 2 & 0 & 0 & 1 & 1 \\ 0 & 0 & 0 & 1 & 0 & 1 & 1 & 2 & 1 & 1 & 0 & 0 \\ 0 & 0 & 1 & 1 & 0 & 1 & 1 & 4 & 1 & 1 & 1 & 1 \\ 0 & 0 & -1 & 0 & -1 & -2 & 0 & -1 & 0 & 0 & -1 & -1 \\ 0 & 1 & 1 & 0 & 1 & 2 & 0 & 1 & 0 & -1 & 1 & 1 \\ 0 & 0 & 1 & 0 & 0 & 0 & 0 & 1 & 0 & 1 & 1 & 1 \end{pmatrix}} = \textbf{UAC}$$

5. und 12. Spalte von UAC werden getauscht, damit Det. $A_{22} \neq 0$. Besonders wegen Verkleinerung des (späteren) Rechenaufwandes erfolgt hier eine "Ordnungserniedrigung" 4) von Ordnung 12 auf Ordnung 6. Unterteilung der Matrix 12. Ordnung in 4 "Untermatrizen" 6. Ordnung: $A_{11}$, $A_{12}$, $A_{21}$, $A_{22}$ bzw. $A_{22}^{-1}$ (inverse Matrix):

$$\left(\begin{array}{cccccc|cccccc} 0 & 0 & 0 & 1 & 1 & 0 & 0 & 0 & 0 & 0 & 0 & 0 \\ 2 & 0 & 1 & 0 & 1 & 1 & 0 & 1 & 0 & 2 & 1 & 1 \\ 0 & 1 & 1 & 2 & 0 & 1 & 1 & 2 & 0 & 0 & 0 & 1 \\ 1 & 0 & 1 & 0 & 0 & 1 & 0 & 0 & 0 & 1 & 0 & 1 \\ 1 & 0 & 1 & 2 & 0 & 3 & 1 & 2 & 1 & 2 & 0 & 1 \\ 0 & 0 & 1 & 1 & 0 & 1 & 1 & 1 & 0 & 0 & 0 & 1 \\ \hline 0 & 0 & 2 & 1 & 1 & 4 & 1 & 2 & 0 & 0 & 1 & 2 \\ 0 & 0 & 0 & 1 & 0 & 1 & 1 & 2 & 1 & 1 & 0 & 0 \\ 0 & 0 & 1 & 1 & 1 & 1 & 1 & 4 & 1 & 1 & 1 & 0 \\ 0 & 0 & -1 & 0 & -1 & -2 & 0 & -1 & 0 & 0 & -1 & -1 \\ 0 & 1 & 1 & 0 & 1 & 2 & 0 & 1 & 0 & -1 & 1 & 1 \\ 0 & 0 & 1 & 0 & 1 & 0 & 0 & 1 & 0 & 1 & 1 & 0 \end{array}\right) \qquad \begin{array}{c|c} A11 & A12 \\ \hline A21 & A22 \end{array} \qquad \mathbf{A_{22}^{-1}} = \begin{pmatrix} 1 & 1 & -1 & 4 & 2 & 2 \\ 0 & -1 & 1 & -1 & -1 & -1 \\ -1 & 2 & -1 & -1 & 1 & 0 \\ 0 & 0 & 0 & -1 & -1 & 0 \\ 0 & 1 & -1 & 2 & 2 & 2 \\ 0 & 0 & 0 & -2 & -1 & -1 \end{pmatrix}$$

Vertauschung

UAC,5. ⟷ 12. Spalte

Für die Entwicklung des Schur-Complements 4) $A_{11,red}$. hat dann folgende "Formelgleichung" Gültigkeit:

$$A_{11,red} = A_{11} - A_{12} * A_{22}^{-1} * A_{21}$$

Das Schur-Complement 4) 6. Ordnung des Codons UAC :

$$\begin{pmatrix} 0 & 0 & 0 & 1 & 1 & 0 \\ 2 & 2 & 0 & 0 & 0 & -1 \\ 0 & 2 & 0 & 1 & 0 & -1 \\ 1 & 2 & 1 & 0 & 0 & -1 \\ 1 & 2 & 1 & 1 & 0 & 0 \\ 0 & 0 & 0 & 0 & 0 & -1 \end{pmatrix}$$

**Die *dritte* Nucleotidbasen des Codons**

Am oben stehenden Musterbeispiel UAC sieht man, dass die *dritte* Nucleotidbase C matrizentheoretisch genauso behandelt wird wie die ersten zwei Nucleotidbasen U und A. Stets gilt die "nichtkommutative" Matrizenmultiplikation. UAC (tyr), UCA (ser), CAU (his), CUA (leu), AUC (ileu), ACU (thr) sind also 6 charakteristische und voneinander *verschiedene* Matrizen, obwohl die "Menge": U, A, C immer dieselbe ist. Nach dem heutigen Kenntnisstand aus den Lehrbüchern und unter Berücksichtigung der *Degeneration* des Genetischen Codes wäre folgende Aussage *kein* Widerspruch: (Beispiel !) In den Codons: GCU, GCC, GCA, GCG, hat die *dritte* Nucleotidbase, genetisch gesehen, *keine* Bedeutung ; in den Codons UGU, UGC, UGA, UGG hat die jeweils *dritte* Nucleotidbase eine enorm genetische Bedeutung. (Letzteres gilt übrigens für insgesamt 12 der 20 Aminosäuren). Die hier angewandte Mathematik lässt also diesen Widerspruch nicht zu, man könnte jetzt sagen: *In rein mathematischer Hinsicht existiert keine Degeneration des Genetischen Codes.* Weiter unten wird die Konsequenz aus dieser Auffassung mitgeteilt.

Das *Codon-Paar.*

Für die ganze Arbeit soll gelten: Die m(essenger)-RNA wird mathematisch als "Codonpaar-Sequenz" formuliert, also *ohne* die "Di-Ribose-Phosphat-Brücken" zwischen den einzelnen Nucleotidbasen des Codons oder Tripletts.

Aus Gründen einer "vorgezogenen" Spezifität (genauere Begründung s. unten) wird mit *Codon-Paaren* gearbeitet. A und B sollen Codons sein. Nach folgender "Formel" wird dann das *Codon-Paar* A - B matrizentheoretisch folgendermaßen mit der Matrix M dargestellt:

$$\boxed{A - B \longrightarrow B^{-1} * A \longrightarrow M}$$

( Die inverse "Codon- Matrix" B, 6. Ordnung, wird multipliziert mit der "Codon-Matrix" A , 6. Ordnung, zu der "Codonpaar-Matrix" M, 6. Ordnung).

Jedes x-beliebige *Codon-Paar* X - Y besitzt eine Matrix Z von 6. Ordnung. Ohne Berücksichtigung von einem "Startcodon", von drei "Stoppcodons", und von 60 "gleichnamigen" *Codon-Paaren* (also z. B. GCU - GCU) existieren absolut 3 5 4 0 *Codon-Paare.* Da aber bei der (folgenden) *Eigenwert-Aufgabe* allgemein die *Codon-Paare* X - Y und Y - X (Codons in sich vertauscht) nach der Rechenvorschrift für Matrizenpaare (s.oben) zueinander *reziproke* Eigenwerte besitzen, sind "nur" 1 7 7 0 "Codonpaar-Matrizen" zu berechnen. Bis jetzt wurden ca. 500 "Codonpaar-Matrizen" ausgerechnet.

**Die EIGENWERT-AUFGABE**

Da man je *Codon-Paar* eine Matrix besitzt, kann mit dieser die spezifische oder einfache *Eigenwert-Aufgabe* 4) durchgeführt werden. (In dieser Publikation wird aus Gründen des Textumfanges auf eine genaue Erklärung des *Eigenwertes,* der "charakteristischen Zahl" , einer Matrix und auf seine physikalische Bedeutung in der Matrizen-bzw. Quantenmechanik verzichtet). Für jede "Codonpaar-Matrix" erhält man 6 *Eigenwerte* mit der hier eingeführten Klassenordnung. Klasse 0 : *6 reelle Eigenwerte; Klasse 1 : 4 reelle Eigenwerte, 1 Paar konjugiert-komplexe Eigenwerte; Klasse* 2 : *2 reelle Eigenwerte, 2 Paar konjugiert-komplexe Eigenwerte; Klasse 3 : 3 Paar konjugiert-komplexe Eigenwerte.*

$$\text{Det.} = \begin{bmatrix} a_{11}-\lambda_n & a_{12} & a_{13} & a_{14} & a_{15} & a_{16} \\ a_{21} & a_{22}-\lambda_n & a_{23} & a_{24} & a_{25} & a_{26} \\ a_{31} & a_{32} & a_{33}-\lambda_n & a_{34} & a_{35} & a_{36} \\ a_{41} & a_{42} & a_{43} & a_{44}-\lambda_n & a_{45} & a_{46} \\ a_{51} & a_{52} & a_{53} & a_{54} & a_{55}-\lambda_n & a_{56} \\ a_{61} & a_{62} & a_{63} & a_{64} & a_{65} & a_{66}-\lambda_n \end{bmatrix} = 0$$

In der (allgemeinen) Matrix 6. Ordnung tritt der *Eigenwert* λ als Subtrahend und "Diagonal-Element" auf.
n = 1 bis 6, ein Polynom n-ten Grades
Det. = Determinantenwert gleich Null.
Symmetrische Matrix: $a_{ki} = a_{ik}$
Nichtsymmetrische Matrix $a_{ik} \neq a_{ki}$

Bei den Eigenwerten spricht man bekanntlich auch von "charakteristischen Wurzeln"; sie sind bei einer reellen und *symmetrischen* Matrix alle reell. Bei den "Codonpaar-Matrizen" handelt es sich ausschließlich um *"nichtsymmetrische"* Matrizen, die sehr oft neben den konjugiert-komplexen Eigenwerten auch reelle Eigenwerte besitzen; hierzu Klassen 0 bis 2. Es soll hier von einer *"Pseudosymmetrie"* gesprochen werden, die später bei der Formulierung von "Codonpaar-Sequenzen" und entsprechenden Aminosäuresequenzen berücksichtigt werden soll.

Der negative Eigenwert

Wenn die "Urmatrix" M auf seinen *Eigenvektor* "wirkt", behält der *Eigenvektor* seine Richtung bei und λ tritt als "Verlängerungs - oder Verkürzungsfaktor" auf wenn er positiv ist. Bei einem *negativen Eigenwert* wird der verlängerte oder verkürzte *Eigenvektor* um $180^0$ gedreht, er besitzt also exakt die Gegenrichtung von vorher. (s. weiter unten: "Polarität")

[Ursprünglich wurde - je *Codon-paar* - mit den 6 *Eigenvektoren* gearbeitet, und zwar mit den *"Euclidean Distances" zwischen* den einzelnen *Eigenvektoren.* Ein gewisser Vorteil bestand darin, dass zwischen *konjugiert-komplexen Eigenvektoren* diese *"Euclidean Distances"* reel l sind, das "Konjugiert-Komplexe" also in die Aufgabenstellung miteinbezogen werden konnte. Mit diesen *"Euclidean Distances"* ("Abstände" im streng mathematischen Sinne sind es nicht) wurde dann ein *Doppelverhältnis* - eine wesentliche *Invariante* der *Projektiven Geometrie* 5)- definiert, um festzustellen, ob auch hier zwischen direkt benachbarten *Codon-Paaren* eine *Invarianz* vorliegt. Trotz anfänglicher und durchaus günstiger Rechenergebnisse, die auch eine mathematische Erweiterung erfahren könnten, wurde dieses spezielle Rechenverfahren mit den *Eigenvektoren* wegen eines enormen Rechenaufwandes nicht weiter verfolgt].

"Codonpaar-Matrizen" mit besonderen Eigenwerten

Es sollen hier zwei allgemeine, aber auch besondere, Beispiele gezeigt werden (hier stark gekürzt). Es wurde stets mit dem Programm: wolfram mathematica home edition gearbeitet. Im Anhang findet man die Schur-Complemente der Codons.

Beispiel: tyr- cys, UAU - UGU.

$$\text{UGU invers mal UAU} = \begin{pmatrix} -4 & 0 & -6 & 0 & 4 & 0 \\ 0 & 4 & 0 & 3 & 3 & 0 \\ 2 & 0 & 4 & 0 & -2 & 0 \\ 2 & -4 & 3 & -2 & -5 & 0 \\ -2 & 0 & -3 & 0 & 3 & 0 \\ -4 & 1 & -6 & 1 & 5 & 1 \end{pmatrix}$$

Eigenvalues [m6x6], Klasse 1

$$\{1+\sqrt{5}, 1+i\sqrt{3}, 1-i\sqrt{3}, 1-\sqrt{5}, 1, 1\}$$

{3.23607,1.+1.73205 I,1.-1.73205 I,-1.23607,1.,1.}

3.236/2=1.618, GS-Zahl

Beispiel: his - leu, CAU -CUU.

Eigenvalues [m6x6], Klasse 0

$$\text{CUU invers mal CAU} = \begin{pmatrix} -1 & 0 & 2 & 0 & 0 & 0 \\ 0 & 4 & 0 & 3 & 3 & 0 \\ \frac{1}{2} & 0 & 0 & 0 & 0 & 0 \\ -1 & 0 & -5 & 1 & 0 & 0 \\ \frac{1}{2} & -2 & 3 & -2 & -1 & 0 \\ 0 & -1 & 3 & -1 & -2 & 1 \end{pmatrix}$$

$$\{2, \frac{1}{2}(-1-\sqrt{5}), 1, 1, 1, \frac{1}{2}(-1 + \sqrt{5})\}$$

Im letzten Fall erhält man die Zahlen des GOLDENEN SCHNITTES 7)

{2., -1.618033988749895,1.,1.,1.,0.6180339887498949}

Auch für das Codon-Paar: UGC-GGC und "Aminosäurepaar" cys-gly, treten die Eigenwerte als "Goldene-Schnittzahlen" auf. Angesichts 1770 "Codonpaar-Matrizen" werden mit hoher Wahrscheinlichkeit noch "etliche" Matrizen auftreten, deren Eigenwerte "Goldene-Schnitt-Zahlen" sind. Es eröffnet sich hier also die Möglichkeit, künstliche Aminosäuresequenzen zu schaffen, wobei die "Abfolge" der einzelnen Aminosäureradikale in Gestalt ihrer *Codon*-Paare -letztendlich und indirekt - durch den *Goldenen Schnitt und seine Zahlen* bestimmt wird (hierzu weiter unten).

Das gleichnamige Aminosäurepaar,

Bei der Eigenwert-Berechnung der *Codon-paare* von gleichnamigen Aminosäurepaaren tritt eine "paradoxe" Situation auf. Beispiele: Das Aminosäurepaar: tyr-tyr wird codiert durch die *Codon-Paare:* UAU-UAC und dem inversen Paar: UAC-UAU. Insgesamt liegen also zwei 6-Eigenwert-Sätze vor, wobei beide zueinander *reziproke* Eigenwerte besitzen. Die Aminosäure *Prolin* kann mit 4 Codons codiert sein: CCU, CCC, CCA, CCG, Insgesamt existieren also für pro-pro 12 *Codon-Paare* mit 72 Eigenwerten, wobei 36 *reziprok* sind zu den anderen 36. (Für die Aufgabenstellung sind es natürlich viel weniger, weil die konjugiert-komplexen Eigenwerte - zunächst wenigstens - nicht benutzt werden). Ein gleichnamiges Aminosäurepaar kann also bei vielen aufzubauenden Aminosäuresequenzen an vielen *verschiedenen Positionen* auftreten bzw. eingesetzt werden (s.a. hier S. 14, Methoden...).

**"Prosymmetrie" und "Nichtsymmetrie" bei Aminosäurepaaren**

Hier oben wurden bereits die *Eigenwertklassen* definiert. Bei 20 Aminosäuren(radikale) existieren $20^2 = 400$ Aminosäurepaare. Die gleichnamigen Aminosäurepaare sind hier enthalten. Jedes dieser 400 Paare kann mittels seiner Codonpaare bzw. Codonpaarmatrizen und damit seiner Eigenwertklassen *vor* jeglicher Formulierung einer Aminosäuresequenz "symmetrisch" charakterisiert werden. Folgendes wird hier definiert: "Symmetrieklasse" 1 bedeutet "Prosymmetrie", Abkürzung: ps, bei der Codonpaarmenge überwiegen die Eigenwertklassen 0 und 1. "Symmetrieklasse" 2 bedeutet "Pseudosymmetrie", Abkürzung: pss, bei der Codonpaarmenge sind zu ca. 50% die Eigenwertklassen 0 und 1 und zu ca. 50% die Eigenwertklassen 2 und 3 vertreten. Man könnte hier auch von einer symmetrischen *Neutralität* sprechen. "Symmetrieklasse" 3 bedeutet "Nichtsymmetrie", Abkürzung: ns, bei der Codonpaarmenge überwiegen die Eigenwertklassen 2 und 3. Eine Unterteilung bei den einzelnen "Symmetrieklassen" ist auch möglich. Beispiele: Das Aminosäurepaar glu - arg: 66.6% Eigenwertklasse 2, 16.6% Eigenwertklasse 1 und 16.6% Eigenwertklasse 0, also Nichtsymmetrie. Das Aminosäurepaar phe - ser: 50% Eigenwertklasse 2, 16.6% Eigenwertklasse 3 und zu 33.3% Eigenwertklasse 1, also Nichtsymmetrie. Das Aminosäurepaar pro - phe: 50% Eigenwertklasse 0 und 50% Eigenwertklasse 1, also Prosymmetrie. (s. auch S. 14). Diese unterschiedliche Verteilung der "Symmetriearten" bei den Codonpaaren ein und desselben Aminosäurepaares hängt damit zusammen, bei welcher Aminosäuresequenz an welchen Positionen das jeweils betreffende Aminosäurepaar später zu stehen kommt (s.a. S. 15).

**Die "wechsetseitige Eigenwerte-Invarianz"**

Das Kernstück der vorliegenden Arbeit liegt in der Feststellung einer "wechselseitigen Eigenwerte-Invarianz" bei "Codonpaar-Sequenzen", die genetisch den natürlichen Aminosäuresequenzen Bradykinin und Humaninsulin entsprechen. Hierzu die Tabellen auf folgenden Seiten. Die "Verkettung" der einzelnen *Codon-Paare* erfolgt durch gleiche Eigenwert-Beträge, wobei im "Normalfall" - Anfang und Ende der Kette ausgenommen - jedes *Codon-Paar* zwei Eigenwerte aus seinem "eigenen" 6-Eigenwerte-Satz für diese "wechselseitige Eigenwerte-Invarianz" zur Verfügung stellt. Mathematisch beobachtet man bei den einzelnen *Codon-Paaren"* eine Art "ambivalentes" Verhalten nach "links" und "rechts" innerhalb ihrer Sequenz. Bis jetzt "sieht es so aus", dass solche natürlichen "Codonpaar-Sequenzen" in mehrfacher Art und Weise formuliert werden können: Mit und ohne *Vorzeichenwechsel* bei diesen "Eigenwerte-Übergängen von *Codon-Paar* zu *Codon-Paar.* Es soll von einer "indirekten und wechselseitigen Eigenwerte-Invarianz" gesprochen werden, wenn stets dieser **zweite** Eigenwert für die "Verkettung" herangezogen wird, und von einer "direkten, wechselseitigen Eigenwerte-Invarianz" - stark ausgeprägt beim Bradykinin - stets nur e i n Eigenwert für die Beschreibung bzw. "Verkettung" ausreicht (s. Tabellen). "Polar" bedeutet dann: *mit Vorzeichenwechsel,* "nichtpolar": *ohne Vorzeichenwechsel* bei den Eigenwerte-Übergängen.

Mit folgendem Schema soll dieser Sachverhalt mit den Eigenwerten nochmals verdeutlicht werden.

**Die "wechselseitige Eigenwerte-Invarianz" bei "Polycodonpaar-Sequenzen". Allgemeines Schema**

*Codon-Paare* sollen mit Großbuchstaben bezeichnet werden: A, B, C, D ..................

Großbuchstaben mit Strich sind dann zueinander "degenerierte" *Codon-Paare* A', B', C'.....

Beispiel: GUC - GAA = A GUG - GAG = A', beide codieren val - glu.

Kleinbuchstaben: a, b, c, d, e, f, g, h, .............. sind reelle Eigenwerte.

Eine "nichtpolare" "Codonpaar-Sequenz" soll sein: Von *Codon-Paar* zu *Codon-Paar* findet bei den Eigenwerte-Übergängen *kein* Vorzeichenwechsel statt, bei einer genetisch entsprechenden, aber "polaren" "Codonpaar-Sequenz" liegt bei den Eigenwerte-Übergängen *ein* Vorzeichenwechsel vor.

Das erste *Codon-Paar* kann (im allgemeinen Fall) frei gewählt werden, später (im speziellen Fall) wird das *erste* Codon das "Startcodon" sein, die (aufzubauende, künstliche) Aminosäuresequenz beginnt dann mit Methionin.

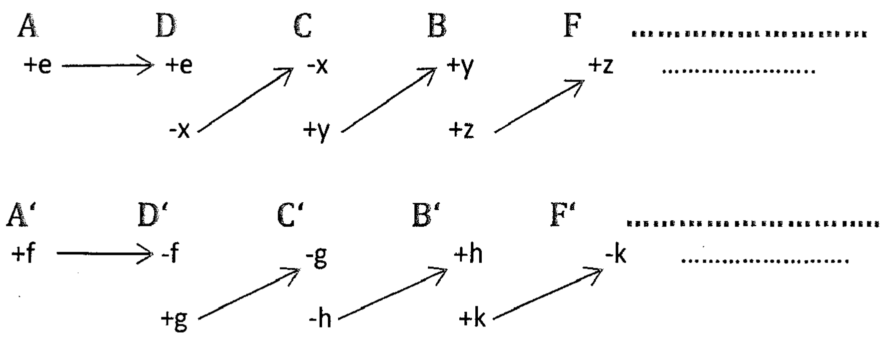


Bei einem *Codon-Paar* handelt es sich stets um zwei (Anfang und Ende der Sequenz ausgenommen) reelle Eigenwerte aus *seinem (eigenem)* 6-Eigenwerte-Satz.

Eine "nichtpolare Codonpaar-Sequenz" sollte stets von einer "polaren" -zur ersten degenerierten - "Codonpaar-Sequenz" begleitet sein (und umgekehrt), damit "Mehrdeutigkeiten" in den Sequenzbildungen eingeschränkt sind. (Sehr oft stehen je Codon-Paar nur zwei reelle Eigenwerte zur Verfügung, also die "Eigenwert-Klasse" 2). Nach relativ vielen Untersuchungen kam man zu dem Schluss, dass eine "Absicherung" der "Abfolge" der *Codon-Paare* auch *innerhalb* einer "Nichtpolarität" bzw. "Polarität" erfolgen kann, vorausgesetzt, dass mehrheitlich *verschiedene* (degenerierte) *Codon-Paare* bei dieser "Sequenzabsicherung" vorliegen (s. Tabellen).

Die "nichtpolare" und "polare" "wechselseitige Eigenwerte- Invarianz" beim Nonapeptid Bradykinin und der A- und B-Kette des Humaninsulins. Tabellen

| **Arg - pro** | **pro - gly** | **phe - ser** | **pro - phe** | **arg** | |
|---|---|---|---|---|---|
| CGU - CCU | CCU - GGU | UUU - UCC | CCG - UUU | CGG | |
| -2.138 → | -2.102 | + 3.536 → | + 3.571 | | |
| | +0.487 → | + 0.5 | - 0.177 → | -0.172 | $PF_m$ = 0.979 |

| **Arg - pro** | **pro - gly** | **phe - ser** | **pro - phe** | **arg** | |
|---|---|---|---|---|---|
| CGU-CCA | CCC - GGC | UUU - UCC | CCG - UUU | CGG | |
| -0.210 → | -0.219 | + 3.536 → | + 3.571 | | |
| | -1.28 → | -1.32 | +0.245 → | +0.240 | $PF_m$ = 0.973 |

| Arg - pro | pro - gly | phe - ser | pro - phe | arg | |
|---|---|---|---|---|---|
| CGG - CCC | CCC-GGU | UUU-UCA | CCC - UUU | CGC | |
| -3.528 | -3.576 | | +0.333 → | +0.373 | |
| | +3.042 → | +3.097 → | +3.095 | | $PF_m$ = 0.964 |

| Arg - pro | pro - gly | phe - ser | pro - phe | arg | |
|---|---|---|---|---|---|
| CGU - CCA | CCU - GGC | UUU - UCU | CCG-UUU | CGG | |
| -2.594 → | -2.674 | -1.597 → | -1.691 | | |
| | +0.259 → | +0.246 | -0.177 → | -0.172 | $PF_m$ = 0.959 |

Eine direkt "polare" Codon-Paar-Sequenz des Bradykinins.

| Arg - pro | pro - gly | phe - ser | pro - phe | arg | |
|---|---|---|---|---|---|
| AGG - CCC | CCA - GGC | UUC - UCG | CCU - UUC | AGA | |
| +0.441 → | -0.446 → | +0.4511 → | -0.447 → | +0.448 | $PF_m$ = 0.9934 |

Stets werden hier die gängigen Abkürzungen für die Aminosäuren (radikale) benutzt. Es wurden also für das Bradykinin insgesamt 60 *Codon-Paare* und die 6 Codons für arg berechnet. Wenn eine "ungerade Sequenzlänge" vorliegt - bei Bradykinin gleich 9, bei der A-Kette des Insulins gleich 21 Aminosäuren (radikale) - wird *ausnahmsweise* zuletzt das *einzelne* Codon für die Berechnung herangezogen (z.B. ergab eine Wiederholung des vorletzten Aminosäureradikals im Sinne der Aufgabenstellung kein günstiges Ergebnis). Bradykinin: (die Zahl in Klammer ist jeweils die Anzahl *Codon-Paare)* arg - pro (24 ), pro-gly (16), phe - ser (12), pro - phe (8), arg (6 Codons). Hier nächste Seite: Die **"wechselseitige Eigenwerte-Invarianz"** beim Humaninsulin. A-Kette: gly - ileu (12), val-glu (8), gln - cys (8), cys - thr (8), ser - ileu (18), cys - ser (12), leu - tyr (12), gln - leu (12), glu - asn (4), tyr - cys (4), asn (2 Codons). B-Kette: phe - val (8), asn - gln (4), his -leu (12), cys - gly (8), ser - his (12), leu - val (24), glu - ala (8), leu - tyr (12), leu - val (24), cys - gly (8), glu - arg (12), gly - phe (8), phe - tyr (4), thr - pro (16), lys - thr (8).

Im rein mathematischen Sinn könnte man bei Vorliegen einer "polaren, direkten bzw. indirekten, wechselseitigen Eigenwerte-Invarianz" sagen: Bei drei aufeinander folgenden *Codon-Paaren* bzw. "Codonpaar-Matrizen" "wirkt" die mittlere Matrix auf einen (direkt) oder zwei (indirekt) ihrer (eigenen) Eigenvektoren *genauso wie* die linke und rechte Matrix auf jeweils einen ihrer (eigenen) Eigenvektoren, und weil dieser "Vorgang" "polar" ist: mit jeweils abwechselnder $180^0$-Drehung des Vektors.

Gly - ile val - glu gln - cys cys - thr ser - ile cys - ser leu - tyr gln - leu glu - asn tyr - cys asn

Die "nichtpolare" A-Kette des Insulins, kein Vorzeichenwechsel bei den Eigenwert-Übergängen, $PF_m$ = Mittelwert der Proportionalitätsfaktoren = 0.981

| GGU-AUU | GUC-GAA | CAA-UGC | UGU-ACA | UCG-AUC | UGU-UCU | UUA-UAC | CAA-CUA | GAA-AAU | UAU-UGU | AAU |
|---|---|---|---|---|---|---|---|---|---|---|
| +2.869 → | +2.934 | -3.942 → | -3.867 | -2.016 → | -1.992 | ( +1 ) | +5.748 → | +5.593 | +3.236 → | +3.002 |
| | -1 → | -1 | -0.124 → | -0.127 | +1 → | +1 → | +1 | +0.988 → | +1 | |

indirekt „nichtpolar" direkt „nichtpolar" indirekt „nichtpolar" $PF_m$ = 0.981 oder 1.019

Die „polare" A-Kette des Insulins, mit Vorzeichenwechsel bei den Eigenwert-Übergängen, $PF_m$ = 0.958 oder 1.043

| GGA-AUC | GUG-GAG | CAA-UGU | UGU-ACG | UCC-AUU | UGC-AGC | UUG-UAC | CAA-CUA | GAA-AAC | UAC-UGU | AAC |
|---|---|---|---|---|---|---|---|---|---|---|
| -7.156 → | +7.337 → | -7.481 → | +6.917 | -1.509 → | +1.504 | +1.207 → | -1.288 → | +1.326 → | -1.106 | |
| | | | -0.171 → | +0.167 | +0.840 → | -0.847 | | | -2 → | +2 |

Direkt „polar" indirekt „polar" direkt „polar"

Phe – val –asn – gln – his – leu – cys –gly – ser – his – leu – val – glu –ala – leu – tyr – leu – val – cys – gly – glu – arg – gly – phe – phe – tyr – thr – pro – lys – thr

Die "nichtpolare" B-Kette des Insulins, kein Vorzeichenwechsel bei den Eigenwert-Übergängen, $PF_m$ = 0.975 oder 1.025

| UUU-GUG | AAC-CAA | CAC-CUG | UGC-GGA | AGU-CAU | CUU-GUA | GAA-GCA | UUG-UAC | UUG-GUG | UGC-GGU | GAG-AGG | GGG-UUU | UUU-UAC | ACC-CCA | AAG-ACU |
|---|---|---|---|---|---|---|---|---|---|---|---|---|---|---|
| +1.234 → | +1.282 | | +1 → | +1 | +1.268 → | +1.202 → | +1.207 | -9.708 → | -9.650 | +2.870 → | +2.727 | -0.5 → | -0.515 → | -0.499 |
| | +0.223 → | +0.227 → | +0.231 | -0.788 → | -0.790 | | +1 → | +1 | +1.512 → | +1.527 | +0.182 → | +0.197 | | |

Direkt „nichtpolar", indirekt „nichtpolar", direkt „nichtpolar" indirekt „nichtpolar" direkt „nichtpolar"

Die „polare" B-Kette des Insulins, mit Vorzeichenwechsel bei den Eigenwert-Übergängen, $PF_m$ = 0.990 oder 1.010 !

| UUU-GUC | AAC-CAA | CAU-CUG | UGC-GGA | AGU-CAU | UUG-GUC | GAA-GCG | CUG-UAU | CUA-GUA | UGC-GGU | GAG-CGC | GGU-UUU | UUU-UAC | ACG-CCA | AAG-ACU |
|---|---|---|---|---|---|---|---|---|---|---|---|---|---|---|
| -1.2614 → | +1.2827 | -1 → | +1 | +1 → | -1 | | -1 → | +1 | +0.4664 → | -0.4614 → | +0.4630 | -0.5 → | +0.5 → | -0.4991 |
| | -3.0345 → | +2.9404 | -1.6536 → | +1.6734 | +2.9239 → | -2.8587 → | +2.8360 | -1.4850 → | +1.5124 | | +1 → | -0.9828 | | |

Indirekt „polar" direkt „polar", indirekt „polar" direkt „polar", indirekt und direkt „polar"

Methoden zur Darstellung hochspezieller Aminosäuresequenzen

*A. Allgemeine Einführung in die Problemstellung*

Im Folgenden ist stets von "Codonpaar-Sequenzen" die Rede, im Grunde meint man damit die m(essenger)-RNA's, die bekanntlich für die genetische Übersetzung in Aminosäuresequenzen - Polypeptide - biochemisch unentbehrlich sind. Die mathematischen Ergebnisse einer matrizentheoretischen Beschreibung von "Codonpaar-Sequenzen", die genetisch *natürlichen* Aminosäuresequenzen entsprechen, eröffnen Möglichkeiten von Darstellungen hochspezieller Aminosäuresequenzen. Es ist vorstellbar, Peptide bzw. Proteine, deren "Codonpaar-Sequenzen" durch x-beliebige, direkte ode indirekte, "polare" oder "nichtpolare" "wechselseitige Eigenwerte-Invarianzen" mittels eines allumfassenden Computerprogramms "zusammengestellt" wurden, mit der Merrifield-Technik oder mit gentechnologischen Verfahren herzustellen. Die Eigenwertbeträge selbst sollen bei den "Eigenwert-Übergängen" im Idealfall bis zur ersten Kommastelle identisch sein. Für jede künstliche Darstellung einer "Codonpaar-Sequenz" könnte vorher ein ganz bestimmter Bereich des Mittelwertes der Quotienten der direkt benachbarten Eigenwertbeträge festgelegt werden, in den Tabellen ist dies der $PF_m$-Wert. Relativ viele "Codonpaar-Matrizen" besitzen (auch mehrfach) den Eigenwert: +1 und -1. Ohne Berücksichtigung der "Abfolge" bzw. Reihenfolge der *Codon-Paare* könnte man dann z.B. eine "Codonpaar-Sequenz" mit der folgenden, direkten und "polaren" "wechselseitigen Eigenwerte-Invarianz" zusammenstellen: +1, -1, +1, -1, +1, -1 etc. Bis jetzt "sieht es so aus", dass der Eigenwert +1 oder -1 bei längeren "Codonpaar-Sequenzen" (s. übersetzt, das Insulin) zur Aufrechterhaltung der weiteren Eigenwerte-Invarianz notwendig ist. Damit jedoch auch die richtige Abfolge der *Codon-Paare* "abgesichert" ist, sollte der jeweils *zweite* Eigenwert des betreffenden *Codon-Paares* keine Eins sein (s. Insulin). Mit dem allumfassenden Computerprogramm soll dann stets dafür gesorgt sein, dass bei der jeweils aufzubauenden "Codonpaar-Sequenz" eine **maximale** "Sequenzlänge" erzielt wird, der Auswahl des jeweils **zweiten** Eigenwertes kommt hier also eine entscheidende Bedeutung zu. Im Kern betrachtet ist jede "Codonpaar-Sequenz" durch den **ersten** Eigenwert des *ersten Codon-Paares* mit seiner maximalen "Sequenzlänge" "vorausbestimmt". Die "Gleichwertigkeit" der dritten Nucleotidbase gegenüber den zwei ersten Nucleotidbasen des Codons sorgt letztendlich dafür, dass "degeneriert-gleiche" *Codon-Paare* an *verschiedenen Positionen* einer (aufzubauenden) Sequenz auftreten können, und somit eine *Wiederholung* eines *gleichen* Aminosäurepaares zulassen. Grundsätzlich kann mit jedem x-beliebigen Aminosäurepaar bzw. Codonpaar der Aufbau einer Codonpaarsequenz begonnen werden. Zunächst soll dieser Beginn nur mit dem Codonpaar: AUG - X (AUG = Startcodon, Methionin, X = beliebiges Codon im Sinne der jeweiligen Aufgabenstellung; insgesamt 60 Paare) erfolgen.

*B. Die Festlegung einer Eigenwertklasse*

Oben wurden die Eigenwerteklassen der Codonpaar-Matrizen bereits erwähnt. Bei der polaren A-Kette des Insulins wird folgende "Klasseneinteilung" der Codonpaare festgestellt: 1, 1, 2, 0, 1, 1, 1, 0, 2, 0, 1. Es findet hier also ein "Klassenwechsel" statt. Es ist vorstellbar, dass auf künstliche Art und Weise eine Aminosäuresequenz geschaffen werden kann, deren Codonpaare ausschließlich *einer* Eigenwertklasse angehören, also z. B.: 1, 1, 1, 1, 1, .... Oder: 2, 2, 2, 2, 2, 2, ..........Stets muss also darauf geachtet werden, dass die Eigenwerteinvarianz immer mit *derselben* Eigenwertklasse zustande kommt, die Auswahl des direkt folgenden Codonpaares ist davon abhängig (wäre bei ein und demselben Aminosäurepaar, aber bei einem Codonpaar einer anderen Klasse als vorher festgelegt, eine Invarianz gegeben, dann würden andere Eigenwerte desselben Codonpaares das direkt folgende Codonpaar und damit ein "falsches" Aminosäurepaar auswählen). Dieses Vorgehen bedeutet letztendlich eine gewisse Einschränkung des Genetischen Codes bzw. der Degeneration desselben, die Pro- bzw. Nichtsymmetrie (hier S.) "steuert" sozusagen die Auswahl des jeweils nächsten Codonpaares und damit des Aminosäurepaares, die ganze Sequenz hindurch. Es besteht dann wieder die Frage, ob eine derart aufgebaute Aminosäuresequenz eine *besondere* biochemische Funktion aufweist.

*C. Aminosäuresequenzen mit Berücksichtigung symmetrischer Aspekte.*

Oben wurden bereits die "Symmetrieklassen" von Aminosäurepaaren definiert. Ohne eine "Vorausauswahl" bei den Aminosäurepaaren zu treffen, war man bemüht, allgemein und speziell (Festlegung einer Eigenwertklasse) die Voraussetzungen für eine wechselseitige Eigenwerteinvarianz zu finden. Die Einordnung der 400 Aminosäurepaare in diese Symmetrieklassen macht es jetzt möglich, Codonpaarsequenzen bzw. Aminosäuresequenzen mit Aminosäurepaaren *einer* Symmetrieklasse zu suchen. Da in "relativ vielen" Codonpaarmengen der Aminosäurepaare *jede* Eigenwertklasse vorkommt, ist es durchaus vorstellbar, dass man ns- bzw. ps- Aminosäurepaare mit wechselseitiger Eigenwerteinvarianz und *einer* festgelegten Eigenwertklasse "verketten" kann. Bei nicht festgelegter Eigenwertklasse kann eine paradoxe Situation eintreten: ns- bzw. ps-Aminosäurepaare werden durch wechselseitige Eigenwerteinvarianz und Codonpaare "verkettet", wobei einige der letzteren eine gegensätzliche Symmetrie zu der "Hauptsymmetrie" der Aminosäurepaare aufweisen können. Nach den bisherigen Erfahrungen wird diese "Verkettungsart" "vermischt" auftreten,

stark abgekürzt: $ns_1(1)$-$ns_2(2)$ - $ns_3(2)$ - $ns_4(0)$ -........ , oder: $ps_1(2)$ - $ps_2(0)$ - $ps_3(1)$ - $ps_4(1)$ - $ps_5(2)$ -.............Die Zahl in Klammer ist jeweils die Eigenwertklasse des Codonpaares für die gemeinsame Eigenwerteinvarianz. (Mit den Indices soll nur gezeigt werden, dass es sich um *verschiedene* Aminosäurepaare handelt).

*D. Die Permutationsmethode*

Bei einer direkten, wechselseitigen, polaren bzw. nichtpolaren Eigenwerteinvarianz (s. S. 9) wird die "Verkettung" der Codonpaare durch einen konstanten Eigenwertbetrag vermittelt. Wenn z.B. diese "Verkettung" mit dem Eigenwert +/- 2 durchzuführen ist, dann besteht die Aufgabe, für jedes hier in Frage kommende Codonpäar die richtige Position für die aufzubauende Codonpaarsequenz zu finden, weil sich bei polarer bzw. nichtpolarer Eigenwerteinvarianz das Vorzeichen (abwechselnd) ändert bzw. gleich blesibt. Wegen des stets wechselnden Vorzeichens und der Festlegung des ersten Codonpaares soll mit der Permutationsmethode zunächst eine gewisse Ordnung geschaffen werden, um dann anschließend mit einer stellenweise auftretenden indirekten Eigenwerteinvarianz die Codonpaarsequenz "abzusichern". Mit Anwendung der Regeln der Kombinatorik hierzu ein einfaches Beispiel: Bei 4 Elementen - hier 4 Codonpaare mit den Eigenwerten: +2, -2, +2, -2 - gibt es dann absolut 4! = 24 Permutationen, darunter sind 8 Permutationen mit ständigem Vorzeichenwechsel. Wenn nun noch das erste Codonpaar - hier gleich a (s. unten) - mit seinem Vorzeichen festgelegt werden soll, dann gibt es bei nachfolgend beliebiger "Vorzeichenfolge" nur noch 6 Permutationen: abcd, abdc, acbd, acdb, adbc, adcb, mit a= +, b= -, c= +, d= -; und wegen des notwendigen Vorzeichenwechsels sind von absolut 24 Permutationen nur 2 Permutationen übrig geblieben, die hier in Frage kommen: abcd und adcb, mit + - + - .Wenn auf diese Art und Weise schon eine gewisse Ordnung gefunden wurde, dann muss versucht werden, ob mit anderen, jeweils degenerierten Codonpaaren und mit eine indirekten Eigenwerteinvarianz eine "Permutationssequenz" bestätigt wird. Beim *Insulin* liegt der Schwerpunkt der "Verkettung" auf der Seite der indirekten Eigenwerteinvarianz. Im oben stehenden einfachen und allgemeinen Beispiel liegt der Schwerpunkt auf der Seite der direkten Eigenwerteinvarianz , bzw. man ging von dieser aus. Damit soll gesagt werden, dass eine "Vermischung" von direkter und indirekter Eigenwerteinvarianz möglich ist, und dass *eine* "Verkettungsart" nicht unbedingt für die ganze Sequenz vorliegen muss. Für die "Absicherung" im oben stehenden allgemeinen Beispiel genügt es also, wenn nur stellenweise eine indirekte Eigenwerteinvarianz vorliegt. Wenn von Anfang an eine Codonpaarsequenz mit indirekter Eigenwerteinvarianz gesucht werden soll, ist eine "Vorausauswahl" der Codonpaare mit der Permutationsmethode ungeeignet, besonders deshalb, weil mit Eigenwertpaaren und unregelmäßigem Vorzeichenwechsel gearbeitet werden muß. Dies alles soll dann mit dem "allumfassenden" Computerprogramm bewerkstelligt werden. Zum Schluss dieses Abschnittes soll noch daran erinnert werden, dass die Sache mit den Permutationen stets eine "Vollerhebung" ist, irgendeine "Permutationssequenz" wird dann auch schließlich die gewünschte Codonpaarsequenz sein.

**E. *Aminosäuresequenzen und die Zahlen des Goldenen Schnittes***

Im Folgenden soll "Goldener Schnitt" mit GS abgekürzt werden. Angesichts 3540 *Codon-Paaren* wird es wahrscheinlich sein, dass etliche "Codonpaar-Matrizen" Eigenwert*beträge* besitzen, die auch GS-Zahlen sind. Die Berücksichtigung von **allen** 3540 *Codon-Paaren* beinhaltet letztendlich auch eine *Wiederholung* von Aminosäurepaaren. Sehr wahrscheinlich wird mit den GS-Zahlen Folgendes möglich sein: Man schafft sich eine "Codonpaar-Sequenz" mit einer "polaren",direkten, "wechselseitigen Eigenwerte-Invarianz" mit der wobei je *Codon-Paar* (Anfang und Ende der Sequenz miteinbezogen) e i n Eigenwert eine spezielle GS-Zahl sein soll, hauptsächlich kommen hier +/- 0.618 und +/- 1.618 in Frage. Man schafft sich zunächst eine Folge der Codonpaare mit je einer gleichen GS-Zahl (als Eigenwert) und mit ständigem Vorzeichenwechsel (wie oben beispielhaft beschrieben). Dann werden mit der *Permutationsmethode* alle möglichen Permutationen mit Vorzeichenwechsel ermittelt und es wird versucht, eine oder mehrere Permutationen *indirekt* "abzusichern". Bei einer *nicht*polaren, direkten Eigenwerte-Invarianz gestaltet sich der Aufbau einer Codonpaarsequenz etwas schwieriger, weil die "Vorausauswahl" von Codonpaaren mit Vorzeichenwechsel entfällt, parallel hierzu muss jeweils eine indirekte "Absicherung" gesucht werden. Das Vorhandensein einer "GS-Sequenz" mit einer indirekten "Absicherung" ohne GS-Zahlen würde die ganze Sache nicht "verallgemeinern", weil das Aussuchen der einzelnen *Codon-Paare* bzw. die Abfolge unmittelbar durch die *eine* GS-Zahl als Eigenwert bestimmt wurde, nach einer Permutation, wie oben beschrieben. Man hätte also *letztendlich* eine Aminosäuresequenz in der Hand, die über "Umwege" mit dem *Goldenen Schnitt* zu tun hat, und bei der die "Abfolge", die genaue Reihenfolge, der Aminosäurepaare eine *charakteristische* wäre.

Zusammenfassend kann man sagen: Die "innere, mathematische Struktur" des links stehenden *Codon-Paares* A sucht sich ein *Codon-Paar* B mit mathematisch "ähnlicher" Struktur, und letzteres besitzt dann auch eine "ähnliche" Struktur mit dem rechts stehenden *Codon-Paar* C. Es besteht dann die "Teil-Sequenz" -A-B-C-, und jedes weitere Codonpaar muss mit dem jeweils vorhergehenden Paar (hier C) diese mathematisch "ähnliche" Struktur haben etc. Die jeweils charakteristische "Sequenzlänge" ist erreicht bzw. liegt dann vor, wenn absolut kein *Codon-Paar* für eine "Sequenzverlängerung" mehr zur Verfügung steht. Wenn letztendlich eine künstliche "Codonpaar-Sequenz" mit "direkter

oder indirekter, polarer oder nichtpolarer, wechselseitiger Eigenwerte-Invarianz aufgestellt bzw. aufgefunden wurde, dann ist die Erkenntnis offensichtlich, dass die Mathematik und der damit verbundene, relativ große Rechenaufwand "nur" dazu diente - nach genetischer Übersetzung - eine "höchstspezielle" Abfolge oder genaue Reihenfolge der Aminosäureradikale zu schaffen.

**"Genfehler"**?

Angesichts der matrizentheoretischen Ergebnisse bei den "Codonpaar-Sequenzen" des Nonapeptids Bradykinin und der A- und B-Proteinketten des Humaninsulins stellt sich hier die Frage, ob das Fehlen bzw. die "Unterbrechung" einer "wechselseitigen Eigenwerte-Invarianz bei *natürlichen* Aminosäuresequenzen bzw. deren "Codonpaar-Sequenzen" auf *"Genfehler"* zurückgeführt werden könnte. Für den Organismus "gefährliche" Proteine (Alzheimer, Krebs, AIDS) müssten dann genetisch in alle nur möglichen "Codonpaar-Sequenzen" (Computerprogramm) übersetzt werden, um festzustellen, welche Aminosäureradikale (in Gestalt ihrer Codons) an welchen Sequenzpositionen die "wechselseitige Eigenwerte-Invarianz" "stören" bzw. unterbrechen. Darüber hinaus könnten dann Überlegungen angestellt werden, welche anderen" Aminosäureradikalpaare" - in Gestalt ihrer Codonpaare - notwendig wären, eine "unterbrochene, wechselseitige Eigenwerte-Invarianz" wiederherzustellen.

Die Adjazenzmatrizen von Uracil und Thymin sind hier mathematisch identisch. Bekanntlich findet sich allgemein Thymin als Bestandteil der DNA, es wäre also auch vorstellbar, den "codogenen" Strang der "Doppelhelix" DNA - bei genauer Kenntnis der einzelnen Gen-Abschnitte und der Introns - mit vorliegendem Rechenverfahren direkt zu berechnen.

**Eigenwerte**, **Balmer-Formel, Zahlen des Goldenen Schnittes**

Eine wirklich überraschende Erkenntnis gewinnt man, wenn die Eigenwerte der *Codon-Paare* bzw. "Codonpaar-Matrizen" mit der Balmer'schen Zahlenbeziehung und den Zahlen des Goldenen Schnitts in einen mathematischen Zusammenhang gebracht werden.

Die klassische und modifizierte Balmer-Formel [6)]: Die klassische Balmer-Formel für die Feststellung der Wellenlängen $\lambda$ der Linien des Wasserstoffspektrums lautet: $\lambda = k\ .\ n^2 / (n^2 - m^2)$ mit k = 4 c / R (R = Rydberg-Frequenz, c = Lichtgeschw.). In der klassischen Balmer-Formel ist m konstant gleich 2, in der später modifizierten Formel sind n und m ganze Zahlen 1,2,3,....7,8,9, und n ist stets größer als m. Nur der Term: $n^2 / n^2 - m^2$, die "Balmersche Zahlenbeziehung", soll hier von Interesse sein, für k wird eine Goldene Schnitt-Zahl eingesetzt. Diese Zahlenbeziehung, bestehend aus *ganzen* Zahlen, wird rein mathematisch benutzt für Versuche, die Eigenwerte der "wechselseitigen Eigenwerte-Invarianz" mit Zahlen des Goldenen Schnitts in einen Zusammenhang zu bringen. Es soll nur mit den Eigenwert*beträgen* gerechnet werden: Am direkt polaren Anfang der A-Kette des Insulins (hier Tabellen) stehen die Beträge 7.156, 7.337, 7.481, 6.917, der Mittelwert ist: 7.222. Zunächst wird mit der "GS-Zahl" 2.618 der Proportionalitätsfaktor x ermittelt: 2.618 x = 7.222, x = 2.758. Nun versucht man mit dieser (allgemeinen) "Balmerschen Zahlenbeziehung" 2.75 zu bekommen, am besten ist das Ergebnis:

$[\,5^2 / (5^2 - 4^2)] = 25/9 = 2.77$, also gilt: $2.618 \cdot [\,5^2 / (5^2 - 4^2)] = 7.270$ ( Differenz: 0.048!).

Nächstes Beispiel: Man kann auch mit einer anderen Goldenen Schnitt-Zahl arbeiten. Am Anfang der nichtpolaren A-Kette stehen die Beträge: 2.869 und 2.934, Mittelwert: 2.901. Mit 1.618 gilt:

$1.618 \cdot x = 2.901$, $x = 1.792$, also 1.8. Es gilt dann: $1.618 \cdot [6^2 / (6^2 - 4^2)] = 1.618 \cdot 1.8 = 2.912$ (Differenz = 0.011!).

Die (allgemeine) Balmer'sche Zahlenbeziehung kann auch *reziprok* angesetzt werden:

$2.618 \cdot [(7^2 - 3^2) / 7^2] = 2.136$ CGU – CCU, Bradykinin; $1.618 \cdot [(6^2 - 5^2) / 6^2] = 0.494$ CCU – GGU, Bradykinin.

Sämtliche Eigenwerte der "polaren" B-Kette des Insulins (s. Tabelle) können folgendermaßen mit Zahlen des Goldenen Schnitts und der (allgemeinen) Balmer'schen Zahlenbeziehung ausgedrückt werden (und die Zahl 1 gehört

auch zum Goldenen Schnitt!):

$$\mathbf{0.618} \cdot [(7^2/7^2-5^2)];\ \mathbf{1.618} \cdot [(6^2/6^2-4^2)];\ \mathbf{1.618} \cdot [(10^2/10^2-2^2)];\ \mathbf{1.618} \cdot [(6^2/6^2-4^2)];\ \mathbf{1} \cdot [(7^2/7^2-4^2)];\ \mathbf{0.382} \cdot [(7^2/7^2-3^2)];$$

$$\mathbf{0.382} \cdot [(6^2/6^2-3^2)].$$

**Zusammenfassung und Ausblick**

Die vier Nucleotidbasen *Adenin, Cytosin, Guanin* und *Uracil* werden mit den Zahlen 1 und 0 als *Adjazenzmatrizen* 12. Ordnung dargestellt. Ohne eine mathematische Berücksichtigung der "Di-Ribose-Phosphat-Brücken" zwischen den einzelnen Nucleotidbasen, werden mit der (doppelten) "nichtkommutativen Matrizenmultiplikation" ("Zeilen mal Spalten") die "Codon-Matrizen" 12. Ordnung berechnet, und dabei hat jeweils die *dritte* Nucleotidbase mathematisch denselben" Stellenwert" wie die zwei ersten Nucleotidbasen des Codons. Um einen größeren Rechenaufwand zu vermeiden, werden in einer "Ordnungserniedrigung" - nach dem Mathematiker Schur - die 64 "Codon-Matrizen" 12. Ordnung in die "Schur-Complemente" 6. Ordnung "umformuliert". Es wird mit *Codon-Paaren* bzw. mit "Codonpaar-Matrizen" gearbeitet, damit von Anfang an die mathematische Beschreibung von "Codonpaar-Sequenzen" eine *spezifische* ist. Es werden jeweils 2 Schur-Complemente matrizentheoretisch zu **einer** "Codonpaar-Matrix" kombiniert, damit die Allgemeine Eigenwert-Aufgabe für Matrizenpaare auf die einfache oder klassische Eigenwert-Aufgabe zurückgeführt werden kann. Für jede "Codonpaar-Matrix" existiert ein "6-Eigenwerte-Satz". Trotz "Nichtsymmetrie" dieser Matrizen sind neben konjugiert-komplexen Eigenwerte fast immer auch *reelle* Eigenwerte vorhanden, mit denen hier hauptsächlich gearbeitet wird.(Bei ca. 400 "nichtsymmetrischen Codonpaar-Matrizen" fand man bis jetzt ca. 6 Codon-Paare der "Eigenwert-Klasse" 3). Angesichts von 3540 *Codon-Paaren* ist es sehr wahrscheinlich, dass relativ viele 6- Eigenwert-Sätze existieren, deren "Codonpaar-Matrizen" dieselbe "Pseudosymmetrie" aufweisen.

Das Nonapeptid Bradykinin und das Humaninsulin mit seinen zwei Proteinketten A und B wurden genetisch übersetzt in alle nur möglichen "Codonpaar-Sequenzen". Es stellt sich heraus, dass bei mindestens *zwei* solcher Sequenzen, jeweils eine "wechselseitige Eigenwerte-Invarianz" aufgefunden wird und dies eröffnet für den allgemeinen Fall eine künstliche Darstellung hochspezieller Aminosäuresequenzen. Das *mehrfache* Auftreten von "speziellen, wechselseitigen Eigenwerte-Invarianzen" dient letztendlich der "Absicherung" der Abfolge (genaue Reihenfolge) der Aminosäure (radikale) in der Aminosäuresequenz. Nach Einordnung der Aminosäurepaare selbst in "Symmetrieklassen" bestehen Möglichkeiten für *nur* nichtsymmetrische oder *nur* prosymmetrische Aminosäurepaare, Codonpaarsequenzen zu schaffen, die dann mit ihrer "wechselseitigen Eigenwerte-Invarianz" die genaue Abfolge dieser Aminosäurepaare bestimmen. Es wird auch möglich sein, eine "Codonpaar-Sequenz" zu schaffen, die mit ihren Eigenwertbeträgen "indirekt" mit dem *Goldenen Schnitt* zu tun hat. Darüber hinaus soll untersucht werden, ob bei "Codonpaar-Sequenzen", die von *natürlichen* Aminosäuresequenzen stammen, das Fehlen irgendeiner "wechselseitigen Eigenwerte-Invarianz" auf das Vorhandensein von "Genfehler" zurückzuführen ist. Zum Schluss werden dann alle künstlich formulierten "Codonpaar-Sequenzen" mit "spezifischen, wechselseitigen Eigenwerte-Invarianzen" genetisch übersetzt in die jeweils entsprechenden Aminosäuresequenzen und untersucht, ob derartig konstruierte Proteinketten eine (ausgezeichnete) *biochemische Funktion* "tragen" oder aufweisen.

Zum *Ausblick:* Bei "gefährlichen", biochemisch aktiven, Proteinen bestände die Möglichkeit, herauszufinden, welche "Sequenzpositionen" diese "Gefährlichkeit" ausmachen. Ist letztere auf das Fehlen oder das Vorhandensein einer "spezifisch" stark ausgeprägten "wechselseitigen Eigenwerte-Invarianz" zurückzuführen? Vielleicht ist es auch möglich, festzustellen, ob bei einem biochemisch aktiven Enzym - z. B. der Hydrogenase - "Aminosäureabschnitte" vorliegen, die für die biochemische Aktivität "überflüssig" sind, um damit dann - auf mathematische Art und Weise - den Grund für die Aktivität selbst zu finden. Es eröffnen sich auch Möglichkeiten der Charakterisierung von Stammzellenproteinen. Interessant wäre noch die Beschreibung von Nucleoproteinen oder Viren und die Fragestellung, ob für die Codons des Nucleotid-Anteils auch eine "Eigenwerte-Invarianz" formuliert werden kann.

**LITERATURVERZEICHNIS**

1. Bücher der Biologie, Genetik und Biochemie. Hier besonders: Jacques Monod, Zufall und Notwendigkeit, Philosophische Fragen der modernen Biologie, R. Piper u. Co. Verlag, München, Zürich , 6. Auflage
2. Im Internet: Adjazenzmatrizen. U.a.: Hartmut Ernst, Grundkurs Informatik, Vieweg und Teubner, 4. Auflage
3. Bücher der Matrizenrechnung. Hier besonders: Frank Ayres jr., Matrizen, Theorie und Anwendung, Mc. Graw-Hill Book Company GmbH, Düsseldorf, New York

4. R. Zurmühl und S. Falk, Matrizen und ihre Anwendungen, Teil 1: Grundlagen, Fünfte, überarbeitete und erweiterte Auflage, Springer-Verlag, Berlin Heidelberg New York Tokyo

5. Bücher der Projektiven Geometrie, vor allem: F. Klein, Vorlesungen über nichteuklidische Geometrie, Berlin, Verlag von Julius Springer. R. Courant, H. Robbins, Was ist Mathematik? Dritte Auflage, Springer-Verlag, Berlin, Heidelberg, New York. Louis Locher-Ernst, Projektive Geometrie, Philosophisch-Anthroposophischer Verlag, Goetheanum Dornach/Schweiz, 1980

6. Christian Gerthsen, Lehrbuch der Physik, 9. Auflage, ab S. 390, Springer Verlag, Berlin, Heidelberg, New York 1966. R. Brdicka, Grundlagen der Physikalischen Chemie, VEB Deutscher Verlag der Wissenschaften Berlin 1967

7. Bücher über den Goldenen Schnitt, z.B.: H.E. Timerding, Der Goldene Schnitt, 3. Auflage, Leipzig/ B.G. Teubner/ Berlin 1929. Hans Walser, Der Goldene Schnitt, 2. Auflage, B.G. Teubner, Verlagsgesellschaft Stuttgart, Leipzig, Zürich 1996

# Anhang

## Die Schur-Complemente der 64 Codons

8. UUU, phe

$$\begin{matrix} 0 & 0 & 0 & -\frac{1}{2} & -\frac{1}{2} & 0 \\ 0 & 0 & \frac{3}{2} & 0 & 0 & \frac{1}{2} \\ 0 & \frac{3}{2} & 0 & 2 & \frac{5}{2} & 0 \\ \frac{1}{2} & 0 & 2 & 0 & 0 & \frac{1}{2} \\ \frac{1}{2} & 0 & \frac{5}{2} & 0 & 0 & 1 \\ 0 & \frac{1}{2} & 0 & \frac{1}{2} & 1 & 0 \end{matrix}$$

UUC, phe

$$\begin{matrix} 1 & 0 & 0 & 0 & 0 & 0 \\ 0 & 0 & 1 & 1 & 1 & 1 \\ -3 & -1 & 0 & 0 & 0 & -1 \\ 1 & 0 & 0 & 1 & 1 & 1 \\ 1 & 0 & 0 & 2 & 1 & 1 \\ 0 & 0 & 0 & 0 & 0 & -1 \end{matrix}$$

UUA, leu

$$\begin{matrix} 0 & -\frac{2}{3} & 0 & -\frac{1}{3} & 0 & \frac{1}{3} \\ 1 & \frac{5}{6} & 1 & \frac{7}{6} & \frac{3}{2} & -\frac{1}{6} \\ 0 & \frac{7}{6} & 0 & \frac{5}{6} & \frac{1}{2} & -\frac{11}{6} \\ 2 & \frac{5}{6} & 0 & \frac{7}{6} & \frac{3}{2} & -\frac{1}{6} \\ 2 & \frac{5}{6} & 0 & \frac{13}{6} & \frac{3}{2} & \frac{5}{6} \\ 0 & \frac{1}{6} & 0 & -\frac{1}{6} & \frac{1}{2} & -\frac{5}{6} \end{matrix}$$

UUG, leu

$$\begin{matrix} 0 & \frac{1}{2} & 0 & -\frac{1}{2} & \frac{1}{2} & -1 \\ 0 & 0 & 1 & 1 & 1 & 1 \\ 0 & -1 & 0 & 2 & -2 & 5 \\ \frac{1}{2} & 0 & \frac{3}{2} & 1 & 1 & 1 \\ \frac{1}{2} & 0 & \frac{3}{2} & 2 & 1 & 2 \\ 0 & -\frac{1}{2} & 0 & \frac{1}{2} & -\frac{1}{2} & 2 \end{matrix}$$

UCU, ser

$$\begin{matrix} 0 & 0 & 0 & 1 & 0 & 0 \\ 2 & -3 & 0 & 0 & 0 & 0 \\ 0 & 0 & 2 & 0 & 0 & 1 \\ 1 & -2 & 0 & 0 & 0 & 0 \\ 1 & -2 & 1 & 0 & 0 & 0 \\ 0 & 0 & 1 & 0 & 1 & 0 \end{matrix}$$

UCC, ser

$$\begin{matrix} 0 & 0 & 0 & 1 & 0 & 1 \\ 0 & 0 & -1 & 0 & 0 & 0 \\ 1 & -\frac{1}{2} & 0 & 1 & \frac{3}{2} & 0 \\ 1 & -1 & 1 & 0 & 1 & 0 \\ 3 & -2 & 1 & 0 & 3 & 0 \\ 1 & -\frac{1}{2} & 1 & 0 & \frac{3}{2} & 0 \end{matrix}$$

UCA, ser

$$\begin{matrix} -1 & 0 & -2 & 2 & -1 & -1 \\ 2 & 0 & 0 & 0 & 0 & 0 \\ -\frac{1}{2} & \frac{3}{2} & 0 & 1 & -\frac{1}{2} & \frac{1}{2} \\ 1 & -1 & 1 & 0 & 0 & 0 \\ 0 & 1 & 0 & 0 & 0 & 0 \\ -\frac{1}{2} & \frac{1}{2} & 0 & 0 & -\frac{1}{2} & -\frac{1}{2} \end{matrix}$$

UCG, ser

$$\begin{matrix} 0 & 0 & 0 & 1 & 0 & 0 \\ 2 & -3 & 0 & 0 & -3 & 0 \\ 0 & 0 & 1 & 1 & 0 & 1 \\ 1 & -2 & 0 & 0 & -2 & 0 \\ 1 & -2 & 2 & 0 & -1 & 1 \\ 0 & 0 & 1 & 0 & 0 & 0 \end{matrix}$$

UAU, tyr

$$\begin{matrix} 0 & 2 & 0 & 1 & 1 & 0 \\ 2 & 0 & 3 & 0 & -3 & 0 \\ 0 & -1 & 2 & 0 & 0 & 1 \\ 1 & 0 & 2 & 0 & -2 & 0 \\ 1 & 1 & 2 & 0 & -4 & 1 \\ 0 & 0 & 1 & 0 & 1 & 0 \end{matrix}$$

UAC, tyr

$$\begin{matrix} 0 & 0 & 0 & 1 & 1 & 0 \\ 2 & 2 & 0 & 0 & 0 & -1 \\ 0 & 2 & 0 & 1 & 0 & -1 \\ 1 & 2 & 1 & 0 & 0 & -1 \\ 1 & 2 & 1 & 1 & 0 & 0 \\ 0 & 0 & 0 & 0 & 0 & -1 \end{matrix}$$

UAA, Stopp-Codon

$$\begin{matrix} 0 & -\frac{5}{2} & 0 & 2 & -\frac{1}{2} & 0 \\ 2 & -\frac{1}{2} & 0 & 0 & \frac{3}{2} & 0 \\ 0 & \frac{1}{2} & 2 & 1 & \frac{5}{2} & 1 \\ 1 & -\frac{1}{2} & 1 & 0 & \frac{3}{2} & 0 \\ 1 & 1 & -1 & 1 & -1 & 1 \\ 0 & -\frac{3}{2} & 2 & 0 & \frac{5}{2} & 0 \end{matrix}$$

UAG, Stopp-Codon

$$\begin{matrix} 0 & 3 & 0 & 1 & 0 & 0 \\ 2 & -3 & 3 & 0 & -2 & 0 \\ 0 & -1 & 1 & 1 & 2 & 1 \\ 1 & -2 & 2 & 0 & -1 & 0 \\ 1 & -3 & 1 & 1 & -3 & 1 \\ 0 & 1 & 1 & 0 & 2 & 0 \end{matrix}$$

UGU, cys

$$\begin{matrix} 0 & 0 & 0 & -\frac{1}{2} & -\frac{1}{2} & 0 \\ 0 & 0 & 0 & 0 & -1 & 0 \\ 0 & 0 & 2 & \frac{1}{2} & \frac{1}{2} & 1 \\ \frac{1}{2} & 0 & \frac{1}{2} & 0 & -1 & 0 \\ \frac{1}{2} & -1 & \frac{1}{2} & -1 & -4 & 1 \\ 0 & 0 & 1 & 0 & 1 & 0 \end{matrix}$$

UGC, cys

$$\begin{matrix} 1 & 0 & 0 & 0 & 0 & 0 \\ 0 & 0 & 1 & 0 & 1 & 2 \\ -3 & -1 & 0 & 1 & 0 & -1 \\ 1 & 0 & 0 & 0 & 1 & 2 \\ 1 & 0 & 0 & 1 & 1 & 3 \\ 0 & 0 & 0 & 0 & 0 & -1 \end{matrix}$$

UGA, Stopp-Codon

$$\begin{matrix} 0 & -\frac{5}{8} & 0 & -\frac{1}{2} & \frac{1}{8} & 0 \\ 0 & \frac{1}{8} & 0 & \frac{1}{2} & -\frac{5}{8} & 0 \\ 1 & \frac{3}{2} & 1 & 3 & \frac{3}{2} & 1 \\ 1 & \frac{1}{8} & -1 & \frac{1}{2} & -\frac{5}{8} & 0 \\ 0 & -\frac{1}{2} & -2 & 1 & -\frac{5}{2} & 1 \\ 1 & \frac{5}{8} & 1 & \frac{1}{2} & \frac{15}{8} & 0 \end{matrix}$$

UGG, try

$$\begin{matrix} 0 & 0 & 0 & -1 & 0 & 0 \\ 0 & 0 & 0 & -1 & -1 & 0 \\ 0 & 1 & 1 & 5 & 2 & 1 \\ \frac{1}{2} & 0 & \frac{1}{2} & -1 & -1 & 0 \\ \frac{1}{2} & 0 & -\frac{1}{2} & -1 & -3 & 1 \\ 0 & 0 & 1 & 1 & 2 & 0 \end{matrix}$$

CUU, leu

$$\begin{matrix} 1 & 0 & 3 & 1 & 1 & 0 \\ 0 & 0 & 1 & 0 & 0 & 0 \\ 2 & 1 & 6 & 2 & 2 & 0 \\ 0 & 1 & 0 & 1 & 2 & 0 \\ 0 & 1 & 0 & 1 & 1 & 0 \\ 0 & 1 & -1 & 1 & 1 & -1 \end{matrix}$$

CUC, leu

$$\begin{matrix} 0 & 0 & 0 & 0 & 0 & 1 \\ -2 & 0 & 1 & -1 & -1 & -1 \\ 0 & 0 & 0 & 1 & 0 & 0 \\ 1 & 0 & 0 & 1 & 1 & 1 \\ -2 & -1 & 0 & -4 & -2 & -3 \\ 2 & 0 & 0 & 1 & 1 & 2 \end{matrix}$$

CUA, leu

$$\begin{matrix} 2 & 2 & 0 & 0 & 0 & -1 \\ 0 & 0 & 1 & 0 & \frac{1}{2} & 0 \\ 4 & 3 & 0 & 0 & 0 & -2 \\ 1 & 1 & 0 & 1 & 1 & 1 \\ 1 & 1 & 0 & 1 & 0 & 0 \\ 1 & 1 & 0 & 1 & 0 & 1 \end{matrix}$$

CUG, leu

$$\begin{matrix} 1 & 0 & 3 & 1 & 2 & -1 \\ 0 & 0 & 1 & 0 & 1 & 0 \\ 2 & 1 & 6 & 2 & 4 & -2 \\ 0 & 1 & 0 & 2 & 1 & -1 \\ 0 & 1 & 0 & 1 & 0 & -1 \\ 1 & 1 & 3 & 2 & 2 & -1 \end{matrix}$$

CCU, pro

$$\begin{matrix} 0 & -1 & 0 & 0 & 0 & 0 \\ \frac{1}{2} & 0 & 0 & 0 & 0 & 0 \\ \frac{1}{2} & -1 & 2 & 0 & -1 & 1 \\ 0 & 0 & 1 & 0 & 0 & 0 \\ 0 & -1 & 1 & -1 & 0 & 1 \\ 0 & -2 & 1 & -2 & 0 & 1 \end{matrix}$$

CCC, pro

$$\begin{matrix} 0 & 1 & 0 & 0 & 0 & 0 \\ 1 & 0 & -1 & 0 & 0 & 0 \\ 0 & 0 & -1 & 0 & 1 & 2 \\ 0 & 0 & 1 & 0 & 2 & 3 \\ 0 & -1 & 0 & -\frac{1}{2} & 0 & 0 \\ 0 & 0 & 0 & 0 & 1 & 2 \end{matrix}$$

CCA, pro

$$\begin{matrix} 0 & 1 & 0 & 0 & 0 & 0 \\ 1 & 0 & -1 & 0 & 0 & 0 \\ -1 & 1 & -1 & 1 & 0 & 1 \\ 0 & -1 & 2 & -2 & 0 & 0 \\ 0 & 0 & 0 & 1 & 1 & 1 \\ 0 & 0 & 0 & 1 & 2 & 1 \end{matrix}$$

CCG, pro

$$\begin{matrix} 0 & -1 & 0 & 0 & 0 & 0 \\ \frac{1}{2} & 0 & 0 & 0 & -\frac{1}{2} & 0 \\ 0 & -3 & 0 & -1 & 1 & 1 \\ 0 & 0 & 1 & 0 & 1 & 0 \\ 0 & -1 & 0 & 0 & 1 & 1 \\ 0 & -2 & 0 & -1 & 2 & 1 \end{matrix}$$

CAU, his

$$\begin{matrix} 0 & -2 & 0 & -1 & -1 & 0 \\ \frac{1}{2} & 0 & 0 & 0 & 0 & 0 \\ 0 & 0 & 0 & 1 & 1 & 0 \\ 0 & 0 & 1 & 0 & 1 & 0 \\ -\frac{1}{2} & 2 & -2 & 2 & 2 & 0 \\ -1 & 3 & -5 & 3 & 4 & -1 \end{matrix}$$

CAC, his

$$\begin{matrix} 0 & 1 & 0 & 0 & -1 & 0 \\ 1 & 0 & -1 & 0 & 0 & 0 \\ 0 & 1 & 0 & 0 & 1 & 0 \\ -1 & 0 & 0 & 1 & 0 & 0 \\ -1 & 0 & -1 & 0 & 0 & 0 \\ -4 & 0 & -4 & 1 & -3 & 1 \end{matrix}$$

CAA, gln

$$\begin{matrix} -2 & \frac{2}{3} & -\frac{2}{3} & -\frac{2}{3} & -\frac{4}{3} & -\frac{1}{3} \\ 1 & 0 & -1 & 0 & 0 & 0 \\ 2 & \frac{4}{3} & \frac{2}{3} & \frac{2}{3} & \frac{4}{3} & \frac{1}{3} \\ 1 & 0 & 1 & 0 & 2 & 1 \\ 1 & \frac{2}{3} & \frac{1}{3} & \frac{4}{3} & \frac{2}{3} & \frac{2}{3} \\ 0 & 1 & 0 & 2 & 1 & 3 \end{matrix}$$

CAG, gln

$$\begin{matrix} 0 & -2 & 0 & -1 & 0 & -1 \\ \frac{1}{2} & 0 & 0 & 0 & -\frac{1}{2} & 0 \\ 0 & 0 & 0 & 1 & 0 & 1 \\ 0 & 0 & 1 & 0 & 2 & 1 \\ -\frac{1}{2} & 2 & -2 & 2 & -\frac{3}{2} & 2 \\ -1 & 3 & -3 & 3 & -1 & 5 \end{matrix}$$

CGU, arg

$$\begin{matrix} 1 & 0 & 3 & 1 & 1 & 0 \\ 0 & 0 & 1 & 0 & 0 & 0 \\ 2 & 1 & 6 & 2 & 2 & 0 \\ 0 & 0 & 1 & 0 & 1 & 0 \\ 0 & 1 & 0 & 1 & 1 & 0 \\ 0 & 2 & -1 & 2 & 3 & -1 \end{matrix}$$

CGC, arg

$$\begin{matrix} -1 & 2 & 0 & -1 & -1 & 0 \\ 0 & 0 & 1 & 0 & 0 & 0 \\ -2 & 3 & 0 & -2 & -2 & 0 \\ 0 & 0 & 1 & 0 & 0 & 1 \\ -1 & 1 & 0 & -1 & 0 & 0 \\ -2 & 2 & 0 & -1 & 1 & 1 \end{matrix}$$

CGA, arg

$$\begin{matrix} -2 & 2 & 0 & 0 & -1 & 2 \\ -1 & 0 & 1 & 0 & 0 & 0 \\ -4 & 3 & 0 & 0 & -2 & 4 \\ 1 & 0 & 1 & 0 & 2 & 0 \\ 0 & 1 & 0 & 1 & 0 & 1 \\ 4 & 2 & 0 & 2 & 3 & 2 \end{matrix}$$

CGG, arg

$$\begin{matrix} 0 & 0 & 0 & 1 & 0 & 0 \\ 0 & 0 & 1 & 0 & 1 & 0 \\ 0 & 1 & 0 & 2 & 0 & 0 \\ -1 & 0 & -2 & 0 & 0 & 0 \\ -1 & 1 & -3 & 1 & -2 & 0 \\ -3 & 1 & -9 & 2 & -5 & -1 \end{matrix}$$

AUU, ileu

$$\begin{matrix} 0 & 1 & 0 & 2 & 2 & 0 \\ -\frac{2}{3} & \frac{5}{6} & -\frac{7}{6} & \frac{5}{6} & \frac{5}{6} & -\frac{1}{6} \\ 0 & 3 & 0 & 4 & 4 & 0 \\ \frac{1}{3} & \frac{5}{6} & \frac{5}{6} & \frac{5}{6} & \frac{11}{6} & -\frac{1}{6} \\ 0 & \frac{1}{2} & \frac{1}{2} & \frac{1}{2} & \frac{1}{2} & \frac{1}{2} \\ -\frac{1}{3} & \frac{13}{6} & -\frac{11}{6} & \frac{13}{6} & \frac{19}{6} & -\frac{5}{6} \end{matrix}$$

AUC, ileu

$$\begin{matrix} 1 & 1 & 0 & -1 & 0 & 0 \\ -\frac{3}{2} & 0 & 1 & 0 & -\frac{1}{2} & 0 \\ 2 & 1 & 0 & -2 & 0 & 0 \\ \frac{3}{2} & 0 & 0 & 0 & \frac{1}{2} & 0 \\ -\frac{3}{4} & 0 & 0 & -\frac{1}{2} & -\frac{3}{4} & -1 \\ -1 & 0 & 0 & 0 & -1 & 0 \end{matrix}$$

AUA, ileu

$$\begin{matrix} 0 & 2 & 0 & 1 & 0 & 0 \\ -3 & 0 & 1 & 1 & -\frac{7}{4} & -4 \\ 0 & 3 & 0 & 2 & 0 & 0 \\ -2 & 1 & 0 & 7 & \frac{5}{4} & -6 \\ 0 & 1 & 0 & 2 & 0 & 1 \\ \frac{1}{2} & 0 & 0 & 3 & \frac{3}{4} & \frac{7}{2} \end{matrix}$$

AUG, met, Start-Codon

$$\begin{matrix} \frac{4}{11} & -\frac{5}{11} & \frac{12}{11} & \frac{14}{11} & \frac{8}{11} & \frac{8}{11} \\ -\frac{6}{11} & \frac{2}{11} & -\frac{7}{11} & \frac{1}{11} & -\frac{1}{11} & -\frac{1}{11} \\ \frac{8}{11} & \frac{1}{11} & \frac{24}{11} & \frac{28}{11} & \frac{16}{11} & \frac{16}{11} \\ \frac{7}{11} & -\frac{6}{11} & \frac{21}{11} & -\frac{3}{11} & \frac{25}{11} & \frac{3}{11} \\ \frac{2}{11} & \frac{3}{11} & \frac{6}{11} & \frac{18}{11} & \frac{4}{11} & \frac{15}{11} \\ \frac{1}{11} & -\frac{4}{11} & \frac{3}{11} & -\frac{13}{11} & \frac{13}{11} & -\frac{9}{11} \end{matrix}$$

ACU, thr

$$\begin{matrix} 0 & -2 & \frac{1}{2} & -1 & 0 & \frac{1}{2} \\ \frac{1}{2} & 0 & 0 & \frac{1}{4} & 0 & 0 \\ 0 & 0 & -\frac{1}{2} & 0 & 0 & -\frac{1}{2} \\ 0 & 0 & 1 & -1 & 0 & 0 \\ \frac{1}{2} & 1 & \frac{3}{2} & \frac{9}{4} & -1 & \frac{1}{2} \\ 0 & 0 & \frac{1}{2} & 0 & 0 & -\frac{1}{2} \end{matrix}$$

ACC, thr

$$\begin{matrix} 0 & 1 & 1 & 2 & 0 & 0 \\ 1 & 0 & -1 & -1 & 0 & 0 \\ 0 & 1 & -1 & 0 & 0 & 0 \\ 0 & 0 & 1 & 2 & 1 & 1 \\ 0 & 0 & 0 & 0 & 1 & 2 \\ 0 & 0 & 1 & 0 & 1 & 1 \end{matrix}$$

ACA, thr

$$\begin{matrix} 0 & 2 & 0 & -1 & -3 & -1 \\ 0 & 0 & -2 & 1 & 1 & 1 \\ 1 & 0 & 1 & 0 & 1 & 0 \\ 1 & -4 & 3 & 0 & 7 & 2 \\ 0 & 0 & 0 & -1 & -1 & -1 \\ 0 & -4 & 2 & 1 & 9 & 3 \end{matrix}$$

ACG, thr

$$\begin{matrix} 0 & -2 & 0 & -3 & 0 & -2 \\ \frac{1}{2} & 0 & 0 & \frac{1}{2} & 0 & \frac{1}{2} \\ 0 & 0 & 0 & 5 & 0 & 4 \\ 0 & 0 & 1 & 0 & 0 & 0 \\ \frac{1}{4} & 1 & \frac{3}{2} & \frac{7}{4} & -\frac{1}{2} & \frac{5}{4} \\ 0 & 0 & 1 & -2 & 0 & -2 \end{matrix}$$

AAU, asn

$$\begin{matrix} 0 & -2 & 0 & -1 & -1 & 0 \\ \frac{5}{12} & \frac{1}{12} & -\frac{1}{12} & \frac{1}{12} & -\frac{1}{6} & \frac{1}{4} \\ 0 & 0 & 2 & 1 & -1 & 2 \\ \frac{1}{3} & -\frac{1}{3} & \frac{4}{3} & -\frac{1}{3} & \frac{5}{3} & -1 \\ -\frac{1}{12} & \frac{19}{12} & \frac{29}{12} & \frac{19}{12} & -\frac{7}{6} & \frac{11}{4} \\ 0 & 0 & 1 & 0 & 1 & 0 \end{matrix}$$

AAC, asn

$$\begin{matrix} 2 & 1 & 2 & -1 & 1 & 0 \\ \frac{7}{9} & 0 & -\frac{11}{9} & \frac{1}{3} & -\frac{4}{9} & 0 \\ -\frac{2}{3} & 1 & -\frac{2}{3} & 1 & -\frac{1}{3} & 0 \\ 1 & 0 & 2 & -1 & 2 & 1 \\ -\frac{13}{9} & 0 & -\frac{13}{9} & \frac{5}{3} & -\frac{8}{9} & 0 \\ \frac{5}{9} & 0 & \frac{14}{9} & -\frac{1}{3} & \frac{10}{9} & 1 \end{matrix}$$

AAA, lys

$$\begin{matrix} 0 & -\frac{1}{5} & 0 & -4 & \frac{3}{5} & -\frac{8}{5} \\ -\frac{1}{5} & 0 & -\frac{7}{5} & -\frac{1}{5} & -\frac{4}{5} & -\frac{1}{5} \\ 0 & \frac{7}{5} & 0 & 2 & -\frac{1}{5} & \frac{6}{5} \\ 4 & \frac{1}{5} & 2 & 2 & \frac{17}{5} & \frac{8}{5} \\ -\frac{3}{5} & \frac{4}{5} & -\frac{1}{5} & \frac{17}{5} & -\frac{4}{5} & \frac{9}{5} \\ \frac{8}{5} & \frac{1}{5} & \frac{6}{5} & \frac{8}{5} & \frac{9}{5} & \frac{6}{5} \end{matrix}$$

AAG, lys

$$\begin{matrix} -\frac{3}{20} & -\frac{11}{5} & 0 & -\frac{7}{4} & \frac{3}{4} & -\frac{11}{20} \\ \frac{5}{12} & 0 & -\frac{1}{3} & \frac{1}{12} & -\frac{3}{4} & \frac{1}{12} \\ \frac{1}{20} & -\frac{3}{5} & 0 & \frac{5}{4} & -\frac{1}{4} & \frac{17}{20} \\ \frac{29}{60} & \frac{1}{5} & \frac{7}{3} & \frac{5}{12} & \frac{9}{4} & \frac{13}{60} \\ \frac{1}{60} & \frac{4}{5} & -\frac{1}{3} & \frac{25}{12} & -\frac{3}{4} & \frac{77}{60} \\ \frac{3}{20} & \frac{1}{5} & 1 & \frac{3}{4} & \frac{5}{4} & \frac{11}{20} \end{matrix}$$

AGU, ser

$$\begin{matrix} 0 & 0 & 1 & 1 & 0 & 1 \\ -\frac{5}{8} & -\frac{1}{8} & 0 & -\frac{1}{8} & -1 & \frac{7}{8} \\ 0 & 0 & 3 & 1 & -2 & 3 \\ \frac{1}{2} & \frac{1}{2} & 1 & \frac{1}{2} & 3 & -\frac{3}{2} \\ -\frac{1}{8} & -\frac{5}{8} & 1 & -\frac{5}{8} & -2 & \frac{11}{8} \\ 0 & 0 & 1 & 0 & 1 & 0 \end{matrix}$$

AGC, ser

$$\begin{matrix} 6 & 1 & 0 & -3 & 2 & 0 \\ 0 & 0 & 1 & 0 & 0 & 0 \\ 12 & 1 & 0 & -5 & 4 & 0 \\ 0 & 0 & 1 & 0 & \frac{3}{2} & 1 \\ 2 & 0 & 0 & 0 & \frac{1}{2} & 0 \\ 2 & 0 & 1 & 0 & \frac{3}{2} & 1 \end{matrix}$$

AGA, arg

$$\begin{matrix} 0 & \frac{7}{3} & 0 & \frac{11}{3} & -\frac{2}{3} & 1 \\ 3 & 0 & 1 & -2 & 3 & -2 \\ 0 & \frac{11}{3} & 0 & \frac{25}{3} & -\frac{4}{3} & 3 \\ -15 & 0 & 1 & 8 & -10 & 8 \\ 2 & \frac{1}{3} & 0 & \frac{2}{3} & \frac{4}{3} & 0 \\ -7 & 0 & 1 & 4 & -4 & 4 \end{matrix}$$

AGG, arg

$$\begin{matrix} \frac{1}{5} & -\frac{1}{5} & \frac{3}{5} & \frac{2}{5} & \frac{2}{5} & -\frac{2}{5} \\ -\frac{11}{20} & \frac{3}{10} & -\frac{13}{20} & \frac{13}{20} & -\frac{1}{10} & \frac{7}{20} \\ \frac{2}{5} & \frac{3}{5} & \frac{6}{5} & \frac{9}{5} & \frac{4}{5} & \frac{1}{5} \\ \frac{1}{2} & -1 & \frac{5}{2} & -\frac{5}{2} & 3 & -\frac{3}{2} \\ -\frac{1}{20} & \frac{3}{10} & -\frac{3}{20} & \frac{23}{20} & -\frac{1}{10} & \frac{17}{20} \\ \frac{1}{10} & -\frac{3}{5} & \frac{13}{10} & -\frac{13}{10} & \frac{11}{5} & -\frac{7}{10} \end{matrix}$$

GUU, val

$$\begin{matrix} 0 & 0 & 0 & -\frac{1}{2} & -\frac{1}{2} & 0 \\ -\frac{1}{2} & 0 & -1 & 0 & 0 & -\frac{1}{2} \\ 0 & 1 & 0 & \frac{3}{2} & \frac{3}{2} & 0 \\ \frac{1}{2} & 1 & 2 & 1 & 2 & \frac{1}{2} \\ -\frac{1}{2} & 1 & -2 & 1 & 1 & -\frac{1}{2} \\ 1 & 1 & 5 & 1 & 2 & 2 \end{matrix}$$

GUC, val

$$\begin{matrix} \frac{1}{2} & 0 & 0 & -1 & -\frac{1}{2} & 0 \\ -\frac{1}{2} & 0 & 1 & 0 & \frac{1}{2} & 0 \\ -\frac{3}{2} & -1 & 0 & 3 & \frac{3}{2} & 0 \\ -\frac{3}{2} & -1 & 0 & 1 & \frac{1}{2} & 1 \\ -1 & -1 & 0 & 3 & 1 & 0 \\ -2 & -1 & 0 & 1 & 0 & 1 \end{matrix}$$

GUA, val

$$\begin{matrix} -4 & -2 & 0 & -5 & -2 & -3 \\ 5 & 2 & 1 & 6 & 3 & 4 \\ 12 & 5 & 0 & 15 & 6 & 9 \\ 14 & 5 & 0 & 15 & 8 & 9 \\ 8 & 3 & 0 & 11 & 4 & 7 \\ 8 & 3 & 0 & 9 & 5 & 5 \end{matrix}$$

GUG, val

$$\begin{pmatrix} -2 & 0 & -6 & -8 & -4 & -7 \\ 0 & 0 & 1 & 1 & 1 & 1 \\ 6 & 1 & 18 & 24 & 12 & 21 \\ 8 & 1 & 24 & 30 & 17 & 27 \\ 4 & 1 & 12 & 17 & 8 & 15 \\ 7 & 1 & 21 & 27 & 15 & 24 \end{pmatrix}$$

GCU, ala

$$\begin{matrix} 0 & 0 & 0 & 1 & 0 & 0 \\ -1 & -3 & -6 & 0 & 3 & 0 \\ 0 & 0 & 1 & 0 & 0 & 1 \\ -1 & -2 & -3 & 0 & 2 & 0 \\ 0 & 1 & 2 & 1 & 0 & 1 \\ 0 & 0 & 1 & 0 & 0 & 0 \end{matrix}$$

GCC, ala

$$\begin{matrix} 0 & -1 & -1 & -1 & -1 & -2 \\ 1 & 2 & -1 & 0 & -1 & -2 \\ 0 & 1 & 0 & 1 & 0 & 0 \\ 0 & 1 & 1 & 0 & 0 & -1 \\ 0 & 1 & 0 & 0 & 0 & 0 \\ 0 & 0 & 1 & 0 & 1 & 1 \end{matrix}$$

GCA, ala

$$\begin{matrix} \frac{1}{3} & -\frac{1}{3} & -\frac{1}{3} & 0 & -\frac{1}{3} & -\frac{2}{3} \\ 2 & 0 & 0 & 0 & 1 & 0 \\ 0 & -1 & 0 & 1 & 2 & 1 \\ 0 & -2 & 0 & 0 & 1 & 0 \\ 0 & 1 & 0 & 0 & 0 & 0 \\ -1 & -1 & -1 & 0 & 0 & 0 \end{matrix}$$

GCG, ala

$$\begin{matrix} 0 & 2 & 0 & 1 & -1 & 0 \\ 0 & -1 & 0 & 0 & -1 & 0 \\ 0 & -1 & 0 & 1 & 0 & 1 \\ -\frac{1}{3} & 0 & 1 & 0 & \frac{2}{3} & 0 \\ 0 & 0 & 0 & 0 & 1 & 0 \\ 0 & 0 & 1 & 0 & 1 & 0 \end{matrix}$$

GAU, asp

$$\begin{matrix} 0 & 2 & 0 & 1 & 1 & 0 \\ \frac{7}{5} & -\frac{3}{5} & 3 & 0 & \frac{3}{5} & -\frac{3}{5} \\ 0 & -1 & 1 & 0 & -1 & 1 \\ \frac{3}{5} & -\frac{2}{5} & 3 & 0 & \frac{7}{5} & -\frac{2}{5} \\ -\frac{2}{5} & \frac{8}{5} & 0 & 1 & -\frac{3}{5} & \frac{8}{5} \\ 0 & 0 & 1 & 0 & 1 & 0 \end{matrix}$$

GAC, asp

$$\begin{matrix} 0 & 0 & 0 & 1 & 0 & 1 \\ 3 & 4 & 1 & 1 & 0 & 0 \\ 1 & 4 & 1 & 2 & 0 & 0 \\ 3 & 5 & 4 & 2 & -1 & 0 \\ 0 & 2 & 0 & 3 & 0 & 1 \\ 1 & 1 & 2 & 1 & -1 & 0 \end{matrix}$$

GAA, glu

$$\begin{matrix} -\frac{9}{10} & -\frac{5}{2} & -\frac{3}{10} & \frac{11}{10} & -\frac{11}{10} & -\frac{9}{10} \\ \frac{11}{5} & 0 & -\frac{3}{5} & \frac{1}{5} & \frac{4}{5} & \frac{1}{5} \\ \frac{3}{5} & 2 & \frac{1}{5} & \frac{8}{5} & \frac{2}{5} & \frac{8}{5} \\ \frac{13}{10} & -\frac{1}{2} & \frac{11}{10} & \frac{3}{10} & \frac{17}{10} & \frac{3}{10} \\ -\frac{9}{10} & -\frac{1}{2} & -\frac{3}{10} & \frac{31}{10} & -\frac{11}{10} & \frac{11}{10} \\ \frac{1}{10} & -\frac{1}{2} & \frac{7}{10} & \frac{1}{10} & \frac{9}{10} & \frac{1}{10} \end{matrix}$$

GAG, glu

$$\begin{matrix} 0 & \frac{5}{2} & -\frac{1}{2} & 1 & -1 & 0 \\ \frac{5}{4} & 0 & 3 & -\frac{3}{4} & \frac{7}{4} & -\frac{3}{4} \\ \frac{1}{4} & -2 & 1 & \frac{5}{4} & \frac{3}{4} & \frac{5}{4} \\ \frac{1}{2} & \frac{1}{2} & \frac{5}{2} & -\frac{1}{2} & \frac{5}{2} & -\frac{1}{2} \\ 0 & \frac{1}{2} & -\frac{1}{2} & 3 & -1 & 2 \\ 0 & \frac{1}{2} & \frac{1}{2} & 0 & 1 & 0 \end{matrix}$$

GGU, gly

$$\begin{matrix} 0 & 0 & 0 & -\frac{1}{2} & -\frac{1}{2} & 0 \\ 0 & 0 & 1 & 0 & 0 & 0 \\ 0 & 0 & 1 & \frac{1}{2} & -\frac{1}{2} & 1 \\ 1 & -1 & 5 & -1 & -1 & 1 \\ 0 & -1 & 2 & -1 & -3 & 2 \\ 0 & 0 & 1 & 0 & 1 & 0 \end{matrix}$$

GGC, gly

$$\begin{matrix} 0 & 0 & 0 & -1 & -1 & 0 \\ 0 & 0 & 1 & 0 & 1 & 0 \\ 0 & -1 & 0 & 4 & 3 & 0 \\ 0 & 0 & 1 & 0 & 1 & 1 \\ 0 & -1 & 0 & 4 & 2 & 0 \\ -1 & 0 & 1 & 0 & 0 & 1 \end{matrix}$$

GGA, gly

$$\begin{matrix} -\frac{1}{3} & -\frac{3}{4} & 0 & -\frac{5}{6} & -\frac{1}{12} & -\frac{1}{6} \\ \frac{1}{3} & \frac{1}{2} & 1 & \frac{1}{3} & \frac{5}{6} & -\frac{1}{3} \\ 1 & \frac{5}{4} & 0 & \frac{7}{2} & \frac{1}{4} & \frac{3}{2} \\ \frac{4}{3} & \frac{3}{4} & 1 & -\frac{1}{6} & \frac{25}{12} & -\frac{5}{6} \\ \frac{2}{3} & \frac{1}{2} & 0 & \frac{11}{3} & \frac{1}{6} & \frac{7}{3} \\ 0 & \frac{1}{4} & 1 & -\frac{1}{2} & \frac{5}{4} & -\frac{1}{2} \end{matrix}$$

GGG, gly

$$\begin{matrix} 0 & 0 & 0 & -1 & 0 & 0 \\ 0 & 0 & 1 & 0 & 1 & 0 \\ 0 & 1 & 0 & 4 & 0 & 1 \\ 1 & 0 & 4 & 2 & 4 & 1 \\ 0 & 1 & 0 & 4 & 0 & 2 \\ 0 & 0 & 1 & 1 & 2 & 0 \end{matrix}$$

*Ende Einschub*

Ein weiterer Aspekt bei der vorliegenden Erfindung ist der Folgende:

Trotz "Nichtsymmetrie" bei den Codonpaar-Matrizen erhält man ja (fast immer) auch reelle Eigenwerte. Es soll dabei folgende Klasseneinteilung gelten:

Klasse 0: 6 reelle Eigenwerte

Klasse 1: 1 Paar konjugiert-komplexe Eigenwerte und 4 reelle Eigenwerte

Klasse 2: 2 Paare konjugiert-komplexe Eigenwerte und 2 reelle Eigenwerte

Klasse 3: 3 Paare konjugiert-komplexe Eigenwerte, kein reeller Eigenwert

Für die "nichtpolare" A-Kette des Insulins findet man: Klasse 1, 2, 1, 1, 1, 2, 1, 0, 1, 1, 1; für die "polare A-Kette: 1, 1, 2, 0, 1, 1, 1, 0, 2, 0, 1.

Also zu 72 % bzw. 54 % der Klasse 1.

Interessant ist es bei der B-Kette, "nichtpolar"': Klasse 2, 1, 1, 0, 1, 1, 1, 1, 1, 0, 0, 1, 0, 1, 0 und "polar": 1, 1, 1, 0, 1, 1, 1, 2, 0, 0, 1, 1, 0, 1, 0.

Sowohl als auch mit 66.6 % Klasse 1 und 33.3 % Klasse Null und 6.6% Klasse 2.

Ein Aminosäurepaar kann in Gestalt seiner (degenerierten) Codonpaare eingeordnet werden in "Nichtsymmetrisch" und "Prosymmetrisch" (Prosymmetrisch bedeutet, dass die Codonpaare vorwiegend Eigenwertklassen 0 und 1 zeigen, Nichtsymmetrisch bedeutet, dass die Codonpaare vorwiegend Eigenwertklassen 2 und höher zeigen, wobei vorwiegend größer und gleich 50% bedeutet.)

Wenn man eine Aminosäuresequenz nur mit "nichtsymmetrischen" Aminosäurepaaren formulieren möchte, dann ist es nicht notwendig, dass die Eigenwert-Invarianz auch immer "Nichtsymmetrisch" ist, mit anderen Worten, es wird keine Eigenwertklasse festgelegt. Dies gilt umgekehrt genauso auch für den Fall der "prosymmetrischen" Aminosäurepaare.

In einer Variante kann die Auswahl der Aminosäuresequenz dadurch erfolgen, dass man die Aminosäurepaare auf Basis von "Nichtsymmetrie", "Prosymmetrie" oder einer Mischung davon auswählt.

In diesem Zusammenhang kann eine Codonpaar-Sequenz zusammengestellt werden mit einer Eigenwerte-Invarianz mit und ohne Vorzeichenwechsel und wobei alle beteiligten Codon-Paare aus derselben Klasse stammen, entweder nur Klasse 0, oder nur Klasse 1, oder nur Klasse 2. (dies nach Umsetzung, wenn möglich ohne Wiederholung von gleichen Aminosäurepaaren, es ist aber keine Bedingung).

Mit dem vorliegenden Verfahren können "Codonpaarsequenzen" aufgebaut werden, die mit dem Codonpaar: AUG-X (X = beliebiges Codon) beginnen und deren sämtliche Codonpaare - auch das *erste* AUG-X - nur *<u>einer</u>* Eigenwertklasse angehören. Bei Berücksichtigung aller nur möglichen Codonpaare mit *derselben* Eigenwertklasse müssen derartige "Codonpaarsequenzen" - mit "wechselseitiger Eigenwertinvarianz" - in ihrer Sequenzlänge "vorausbestimmt" sein. Es kann nun der Fall eintreten, dass an irgendeiner Stelle der (aufzubauenden) "Codonpaarsequenz" *mehrere* Codonpaare - egal, ob diese zu demselben Aminosäurepaar oder zu verschiedenen Aminosäurepaaren gehören - mit *derselben* (festgelegten) Eigenwertklasse für die Fortführung der "wechselseitigen Eigenwertinvarianz" in Frage kommen können. Es finden also "Verzweigungen" statt, die zu *einzelnen* Sequenzen führen. In diesen Fällen wären dann *mehrere* - voneinander verschiedene - "Codonpaarsequenzen" mit *derselben* (zu Beginn festgelegten) Eigenwertklasse möglich, und unter diesen Sequenzen gäbe es dann *eine* oder *mehrere* Sequenzen mit *maximaler* oder *gleicher* Sequenzlänge. Zum Schluss werden dann diese "Codonpaarsequenzen" in die entsprechenden Aminosäuresequenzen übersetzt, synthetisiert, und auf ihre biochemische Funktion hin untersucht. Die wesentlichsten Unterschiede zwischen festgelegter Eigenwertklasse und *nicht* festgelegter, oder "freier" Eigenwertklasse sind: 1. bei festgelegter Eigenwertklasse ist der Einfluss des *ersten* Codonpaares stärker als bei der "freien" Eigenwertklasse. 2. bei festgelegter Klasse sind die Möglichkeiten einer "Sequenzverlängerung" relativ stark eingeschränkt. 3. Aminosäurepaare, deren Codonpaare die einmal festgelegte Eigenwertklasse nicht aufweisen oder besitzen, kommen erst gar nicht zum Einsatz (Vorauswahl vor jeglicher Sequenzierung).

Im Grunde genommen geht es hier darum, Mehrdeutigkeiten "aus der Welt zu schaffen", dass man also zum Schluss eine Aminosäuresequenz besitzt, die wirklich "einmalig" ist.

Bei Klasse 3 ist Folgendes vorstellbar. Bekanntlich sind zwei komplexe Zahlen nur gleich, wenn sowohl der Realteil als auch der Imaginärteil gleich sind.

Bei den Berechnungen im Rahmen der vorliegenden Erfindung (ca. 450 Codonpaare!) ist diese Gleichheit noch nicht festgestellt worden.

Man kann im Rahmen der vorliegenden Erfindung aber Codonpaare suchen, deren komplexe Eigenwerte die gleichen Beträge besitzen, und diese dann mit "wechselseitiger Eigenwerte-Invarianz" zusammenstellen. Dadurch schafft man eine Aminosäuresequenz, deren Ursprung aus einer total nichtsymmetrischen Codonpaar-Sequenz stammt. Da das Vorzeichen bei den Beträgen keine Rolle spielt, hat man dann eine Codonpaar-Sequenz mit "nichtpolarer und wechselseitiger Eigenwert-Beträge-Invarianz" geschaffen und bei Umsetzung gemäß Syntheseverfahren der vorliegenden Erfindung die entsprechende Aminosäuresequenz.

In einer Ausführungsform der vorliegenden Erfindung kann man wie folgt vorgehen:

Bei einer direkten, polaren oder nichtpolaren Zusammenstellung der Codon-Paare - also nur mit einem konstanten Eigenwert, z.B. GS-Zahl: +0.618, - 0.618, +0.618, -0.618 (hier polar) etc. - kann eine indirekte "Absicherung" der Sequenz erfolgen; dies kann dann mit allen möglichen Permutationen überprüft werden. Auch wenn stets mit AUG-X (wobei X für ein beliebiges Codon steht) begonnen wird, ist dies möglich (dann wird mit [n-1]! gerechnet). Es wird in aller Regel dann nur eine ganz bestimmte Permutation (oder zwei, also wenige) geben, die eine indirekte, wechselseitige Eigenwert-Invarianz aufweist. Es besteht eine gewisse Wahrscheinlichkeit, dass nur ganz bestimmte Permutationen biochemisch aktiv sind (beispielsweise ist bekannt, dass eine "Verschiebung" einer Aminosäure ein Enzym inaktiv machen kann). Diese "Permutationsmethode" einer "Absicherung" kann auch bei dem Einsatz der Eigenwerteklasse 3 zur Anwendung kommen, eben weil relativ selten gleiche Eigenwert*beträge* der komplexen Eigenwerte auftreten. Nur die "Nichtsymmetrie" der entsprechenden Codonpaare bestimmt zunächst, welche (übersetzt) Aminosäurepaare überhaupt hier in Frage kommen, und eine Permutation mit Eigenwerte-Invarianz bestimmt dann die genaue Abfolge.

In einer Variante der vorliegenden Erfindung ist die Permutation nur bei direkter Eigenwert-Invarianz anzuwenden.

Damit weist die vorliegende Erfindung eine Reihe von Vorteilen auf:

Es wird durch die vorliegende Erfindung möglich, (mathematisch) "idealisierte" Aminosäuresequenzen zu erstellen.

Daraus resultierend können idealisierte Proteine erstellt werden.

Damit wird das Handwerkzeug an die Hand gegeben, beliebige theoretische Untersuchungen an Aminosäuresequenzen und Proteinen durchzuführen, bei denen der direkte Sequenz/Struktur/Wirkung-Zusammenhang erforscht werden kann.

Es lassen sich leicht (Gen-) Fehler in Aminosäuresequenzen ermitteln und dem folgend deren Zusammenhang mit Krankheiten etc. erforschen.

Ferner lassen sich evolutionäre Zusammenhänge über den Unterschied zwischen den idealisierten Aminosäuresequenzen und den real vorkommenden diskutieren.

Ein weiterer Vorteil der vorliegende Erfindung ist, dass mit ihr in Tumoren Proteine und nichtcodierende RNA untersucht werden und dann Rückschlüsse auf die DNA gezogen werden können.

Weiterhin ist es möglich natürliche Proteine mittels der hier vorgestellten Methode zu überprüfen, ob alle Aminosäuren notwendig sind und - falls nicht - synthetische Proteine zu synthetisieren, welche diese Aminosäuren nicht enthalten, so dass man ein "optimiertes" Protein erhält.

So ist es möglich, mit den erfindungsgemäßen Verfahren Stammzellenproteine zu charakterisieren.

Zur Verdeutlichung der Größenordnungen wird noch angeführt, dass ohne Startcodon und Stopcodons und ohne gleichnamige Codonpaare 3540 Codonpaare existieren. Die "nichtgleichnamigen" Codonpaare für gleichnamige *Aminosäurepaare* und die jeweils inversen, "nichtgleichnamigen" Codonpaare sind in diesen 3540 Codonpaare enthalten. Von den 3540 Codonpaaren müssen aber nur 1770 ausführlich berechnet werden, weil die jeweils inversen Codonpaare auch inverse (reziproke) Eigenwerte besitzen. Beispielsweise sind die 6 Eigenwerte des Codonpaares CCU-CCG reziprok zu den sechs Eigenwerten des Codonpaares CCG-CCU. Weitere Beispiele für das *gleichnamige* Aminosäurepaar: Prolin-Prolin (pro-pro). *Gleichnamige* Codonpaare hierzu sind: CCU-CCU, CCC-CCC, CCA-CCA, CCG-CCG. Die matrizentheoretischen Rechenvorschriften lassen diese Kombinationen nicht zu. Es gibt 12 "nichtgleichnamige" Codonpaare für pro-pro: CCU-CCC, CCU-CCA, CCU-CCG, CCC-CCA, CCC-CCG, CCA-CCG und die sechs inversen hierzu: CCC-CCU, CCA-CCU, CCG-CCU, CCA-CCC, CCG-CCC, CCG-CCA. Diese 12 Codonpaare sind matrizentheoretisch berechenbar. *Gleichnamige* Aminosäurepaare sind pro-pro, arg-arg, ser-ser etc. und *nichtgleichnamige* sind pro- arg, arg-pro, arg-ser, ser-arg etc., womit sich insgesamt 400 Aminosäurepaare ergeben.

In einer Variante der vorliegenden Erfindung ist noch Folgendes möglich:

Die Codonpaarmatrix 6. Ordnung kann nach der Formelgleichung: $A_{11}$,red = $A_{11}$ - $A_{12}.A_{22}$-$^{1}$.$A_{21}$ auf 3. Ordnung "reduziert" werden. Bei Variation des 9. Matrixelementes ($a_{33}$) - in den Berechnungen ist dies b - von minus 10 bis plus 10 erhält man Eigenwerte, die sich auf einer "Kegelschnittlinie" "bewegen" ("befinden"), insbesondere auf einer

Hyperbel. Außerdem wird (zusätzlich) eine rein lineare Abhängigkeit der Eigenwerte beobachtet (Bekanntlich kann - projektiv gesehen- eine Kegelschnitt zu einer geraden Linie "entarten").

Damit erhält man ein Verfahren zur gezielten Herstellung künstlicher Aminosäuresequenzen, indem man

(i) sämtliche 3540 Codonpaarmatrizen 6. Ordnung auf die 3. Ordnung "reduziert", dann
(ii) die Codonpaare, die dasselbe "Hyperbelbild" besitzen mittels Permutation und Eigenwerteinvarianz zu einer Sequenz zusammenstellt, und dann
(iii) diese mittels gentechnologischer Verfahren in die entsprechende Aminosäuresequenz übersetzt.

In einer Variante kann, wenn die Determinante der Untermatrix $A_{22}$ Null ist, auf die Alternativformel $A_{22}$,red = $A_{22} - A_{21}.A_{11}^{-1}.A_{12}$ ausgewichen werden.

Die verschiedenen Ausgestaltungen der vorliegenden Erfindung, z.B. aber nicht ausschließlich diejenigen der verschiedenen abhängigen Ansprüche, können dabei in beliebiger Art und Weise miteinander kombiniert werden.

## Patentansprüche

1. Verfahren zur Synthese hochspezieller Aminosäuresequenzen umfassend die folgenden Schritte:

   a) Erstellung der Adjazenzmatrizen 12. Ordnung von den Nucleotidbasen,
   b) Nichtkommutative Matrizenmultiplikation mit jeweils 3 Adjazenzmatrizen 12. Ordnung
   c) Durchführen einer Ordnungserniedrigung nach Schur mit jeweils einer "Codon-Matrix" 12. Ordnung zur Erstellung von "Codon-Matrizen" 6. Ordnung,
   d) Durchführen der Allgemeinen Eigenwertaufgabe für Matrizenpaare mit jeweils 2 "Codon-Matrizen" 6. Ordnung, wobei je "Codonpaar-Matrix" ein "6-Eigenwerte-Satz" mit 6 reellen und (oder) konjugiertkomplexen Eigenwerten existiert,
   e) bei festgelegtem ersten Codonpaar, in Verbindung mit dem "Startcodon", mit einem reellen Eigenwert für den Aufbau einer Sequenz Suchen von Codonpaaren, die dann eine "wechselseitige Eigenwerte-Invarianz" aufweisen,
   f) genetische Übersetzung der "Codonpaar-Sequenz" in die entsprechende Aminosäuresequenz und
   g) Herstellung der resultierenden Aminosäuresequenz mit fachüblichen Verfahren.

2. Verfahren nach Anspruch 1, **dadurch gekennzeichnet, dass** die Aminosäuresequenz in Schritt g) mittels Merrifield-Synthese hergestellt wird.

3. Verfahren nach Anspruch 1 oder 2, **dadurch gekennzeichnet, dass** im Anschluss an die Synthese die Aminosäuresequenz auf eine biochemische Funktion geprüft wird.

4. Verfahren zur Überprüfung von natürlichen oder synthetisch hergestellten Aminosäuresequenzen auf (Gen)-Fehler bei dem

   Ia) mit einem Verfahren gemäß der Schritte a) bis g) gemäß einem der Ansprüche 1 oder 2 eine Aminosäuresequenz synthetisiert wird,
   II) die zu überprüfende Aminosäuresequenz herangezogen wird,
   III) die beiden Sequenzen verglichen werden und
   IV) anhand der Abweichungen der beiden Sequenzen eventuelle Fehler bestimmt werden,

   wobei die zu überprüfende Aminosäuresequenz entweder eine bereits bekannte Sequenz ist oder aus zu überprüfenden Proteinen mittels Proteinsequenzierung gewonnen wird und
   wobei man das Verfahren gemäß der Schritte a) bis g) gemäß einem der Ansprüche 1 oder 2 mit demselben Aminosäurepaar beginnt, das bei der zu überprüfenden Aminosäuresequenz am Anfang steht.

5. Verfahren zur Überprüfung von natürlichen oder synthetisch hergestellten Aminosäuresequenzen auf (Gen)-Fehler bei dem

   Ib) mit einem Verfahren gemäß der Schritte a) bis f) gemäß Anspruch 1 eine Aminosäuresequenz errechnet wird,
   II) die zu überprüfende Aminosäuresequenz herangezogen wird,

III) die beiden Sequenzen verglichen werden und
IV) anhand der Abweichungen der beiden Sequenzen eventuelle Fehler bestimmt werden,

wobei die zu überprüfende Aminosäuresequenz aus zu überprüfenden Proteinen mittels Proteinsequenzierung gewonnen wird und
wobei man das Verfahren gemäß der Schritte a) bis f) gemäß Anspruch 1 mit demselben Aminosäurepaar beginnt, das bei der zu überprüfenden Aminosäuresequenz am Anfang steht.

**6.** Verfahren nach Anspruch 4 oder 5, **dadurch gekennzeichnet, dass** die zu überprüfende Aminosäuresequenz aus zu überprüfenden Proteinen mittels Edman-Abbau oder De-Novo-Sequenzierung gewonnen wird.

**7.** Verfahren zur gezielten Herstellung künstlicher Aminosäuresequenzen, indem man

(i) sämtliche 3540 Codonpaarmatrizen 6. Ordnung auf die 3. Ordnung "reduziert", dann
(ii) die Codonpaare, die dasselbe "Hyperbelbild" besitzen mittels Permutation und Eigenwerteinvarianz zu einer Sequenz zusammenstellt, und dann
(iii) diese mittels gentechnologischer Verfahren in die entsprechende Aminosäuresequenz übersetzt.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 102014000682


### In der Beschreibung aufgeführte Nicht-Patentliteratur


- Bücher der Biologie, Genetik und Biochemie. Hier besonders: Jacques Monod, Zufall und Notwendigkeit, Philosophische Fragen der modernen Biologie. R. Piper u. Co. Verlag
- Adjazenzmatrizen. U.a. **HARTMUT ERNST.** Hartmut Ernst. Grundkurs Informatik, Vieweg und Teubner
- Bücher der Matrizenrechnung. Hier besonders: Frank Ayres jr., Matrizen, Theorie und Anwendung. Mc. Graw-Hill Book Company GmbH
- **ZURMÜHL ; S. FALK.** Matrizen und ihre Anwendungen. Springer-Verlag
- Bücher der Projektiven Geometrie. Springer-Verlag
- **LOUIS LOCHER-ERNST.** Projektive Geometrie. Philosophisch-Anthroposophischer Verlag, 1980
- **CHRISTIAN GERTHSEN.** Lehrbuch der Physik. Springer Verlag, 1966, 390
- **R. BRDICKA.** Grundlagen der Physikalischen Chemie. VEB Deutscher Verlag, 1967
- **H.E. TIMERDING.** Bücher über den Goldenen Schnitt. Leipzig/ B.G. Teubner, 1929
- **HANS WALSER.** Der Goldene Schnitt. Verlagsgesellschaft, 1996